(19) Europäisches Patentamt
European Patent Office
Office européen des brevets

(11) EP 2 006 284 A1

(12) **EUROPEAN PATENT APPLICATION**
published in accordance with Art. 153(4) EPC

(43) Date of publication:
**24.12.2008 Bulletin 2008/52**

(21) Application number: **07720582.1**

(22) Date of filing: **28.03.2007**

(51) Int Cl.:
***C07D 207/16*** *(2006.01)* ***C07D 275/03*** *(2006.01)*
***A61K 31/426*** *(2006.01)* ***A61P 3/10*** *(2006.01)*
***C07C 49/345*** *(2006.01)*

(86) International application number:
**PCT/CN2007/001008**

(87) International publication number:
**WO 2007/112669 (11.10.2007 Gazette 2007/41)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **05.04.2006 CN 200610066400**

(71) Applicant: **Shanghai Hengrui Pharmaceutical Co. Ltd.**
**Minhang District**
**Shanghai 200-245 (CN)**

(72) Inventors:
- **TANG, Peng Cho**
  **Shanghai 200245 (CN)**
- **LIN, Zhigang**
  **Shanghai 200245 (CN)**
- **ZHANG, Lei**
  **Shanghai 200245 (CN)**
- **WANG, Qian**
  **Shanghai 200245 (CN)**
- **YANG, Jialiang**
  **Shanghai 200245 (CN)**
- **WANG, Yang**
  **Shanghai 200245 (CN)**
- **ZHAO, Fuqiang**
  **Shanghai 200245 (CN)**

(74) Representative: **Ahner, Francis et al**
**Cabinet Régimbeau,**
**20, rue de Chazelles**
**75847 Paris Cedex 17 (FR)**

(54) **DICYCLOOCTANE DERIVATES, PREPARATION PROCESSES AND MEDICAL USES THEREOF**

(57) The present inention relates to new dicyclooctane derivates represented by general formula (I), preparation processes and pharmaceutical compositions containing them, and to uses for treatment especially for dipeptidyl peptidase inhibitor (DPPIV), in which each substituent group of general formula (I) is as defined in specification.

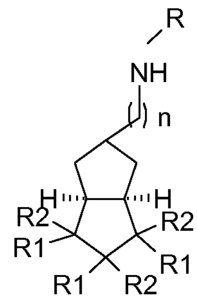

(I)

EP 2 006 284 A1

Printed by Jouve, 75001 PARIS (FR)

## Description

## FIELD OF THE INVENTION

This invention relates to novel bicyclooctane derivatives, methods for their preparation, pharmaceutical compositions containing them and therapeutic use thereof, particularly their pharmaceutical use as dipeptidyl peptidase IV inhibitor.

## BACKGROUND OF THE INVENTION

Diabetes refers to a disease process derived from multiple causative factors and characterized by elevated levels of plasma glucose or hyperglycemia along with sugar, fat and protein metabolism disorder caused by insulin secretion and/or the action defects. Diabetes is an ancient disease, and due to the human body absolute or relative lack of insulin resulting in increased concentrations of glucose in the blood which largely discharges in urine with more drink, more urine, more food, weight loss, dizziness, weakness and other symptoms.

Dipeptidyl peptidase-IV (DPPIV) is a serine protease which cleaves N-terminal dipeptides from a peptide chain containing, preferably, a proline residue in the penultimate position. Although the biological role of DPPIV in mammalian systems has not been completely established, it is believed to play an important role in neuropeptide metabolism, T-cell activation, attachment of cancer cells to the endothelium and the entry of HIV into lymphoid cells (WO98/19998).

More recently, it was discovered that DPPIV is responsible for inhibiting the secretion of glucagon-like peptide (GLP)-1. More particularly, DPPIV cleaves the amino-terminal His-Ala dipeptide of GLP-1, degrading active GLP-1(7-36) $NH_2$ into inactive GLP-1(9-36)$NH_2$ (Endocrinology, 1999, 140: 5356-5363). Under the physiological condition, the half-life of GLP-1 is short, the inactive metabolite from GPP-1 degraded by DPPIV can combine with GLP-1 receptor to antagonize the active GLP-1, so the physiological response to GPL-1 is reduced. The endogenous even exogenous GLP-1 can be entirely protected by the DPPIV inhibitor from being deactivated by DPPIV, and the GLP-1 bioactivity can be significantly increased (5- to 10-fold). Since GLP-1 is a major stimulator of pancreatic insulin secretion and can directly effect on glucose disposal, the DPPIV inhibitor is well useful for treating non-insulin-dependent diabetes mellitus (NIDDM) (US6110949).

## SUMMARY OF THE INVENTION

Accordingly, the present invention relates to compounds of the formula (I) or pharmaceutically acceptable salts thereof:

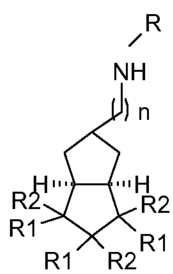

(I)

wherein:

R is selected from the group consisting of alkyl, cycloalkyl, haloalkyl, aryl, heteroaryl, aminocarbonyl alkyl, amide alkyl, heterocyclo aminocarbonyl alkyl and aminoalkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;

$R_1$ and $R_2$ are each independently selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH2)_mC(O)OR4$, $-(CH2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo

alkyl, carboxylic acid and carboxylic ester;

wherein $R_1$ and $R_2$ are attached together with the atom to form a 3 to 8 membered ring, wherein the 3 to 8 membered hetero ring further contains one or more heteroatoms selected from the group consisting of N, O and S, and the 3 to 8 membered hetero rings so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered rings so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_6$ is selected from the group consisting of hydrogen or alkyl;

n is an integral from 0 to 4;

r is an integral from 1 to 6;

m is an integral from 0 to 6.

Further, the present invention includes the compounds of formula (Ia):

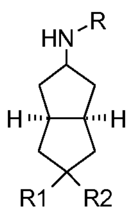

(IA)

wherein:

R is selected from the group consisting of alkyl, cycloalkyl, haloalkyl, aryl, heteroaryl, aminocarbonyl alkyl, amide alkyl, heterocyclo aminocarbonyl alkyl and aminoalkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;

$R_1$ and $R_2$ are each independently selected from the group consisting of hydrogen, hydroxyl, alkyl, cycloalkyl, heterocyclo alkyl, aryl, heteroaryl, $-OR_4$, $-(CH_2CH_2O),R_6$, $-(CH_2)_mC(O)OR_4$, $-(CH2)_mC(O)NR_4R_5$, $-(CH2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

wherein $R_1$ and $R_2$ are attached together with the atom to form a 3 to 8 membered ring, wherein the 3 to 8 membered hetero ring further contains one or more heteroatoms selected from the group consisting of N, O and S, and the 3 to 8 membered hetero rings so formed are each substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together the N atom to form the 3 to 8 membered hetero ring, wherein the 5 to

8 membered hetero ring further contains one or more heteroatoms selected from the group consisting of N, O and S, and the 3 to 8 membered hetero ring so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
r is an integral from 1 to 6;
m is an integral from 0 to 6.

Preferably, in the compounds of the formula (I) or pharmaceutically acceptable salts thereof,
R is heterocyclo aminocarbonyl alkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;
$R_1$ is hydrogen or hydroxyl; $R_2$ is selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH2)_mC(O)NR_4R_5$, $-(CH2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups consisting of alkyl, halogen, aryl, hydroxyl, amino, alkylamino, amide group, alkoxyl, aryloxyl, heterocylco alkyl, carboxylic acid and carboxylic ester;
$R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;
$R_4$ and $R_5$ are each independently selected from the group consisting of hydrogen, alkyl, cycloalkyl, aryl, heteroaryl and heterocyco alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;
meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
r is an integral from 1 to 6;
m is an integral from 0 to 6.

Preferably, in the compounds of the formula (I) or pharmaceutically acceptable salts thereof, R is the formula:

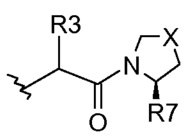

wherein $R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;
$R_7$ is selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heteroalkyl, carboxylic acid, carboxylic ester and halogen;
X is C, S or O.

Preferably, n is O.

In another aspect, this invention provides compounds of formula (I) or pharmaceutically acceptable salts, wherein the compounds of formular (I) are in the pharmaceutically acceptable free-form and the forms of acid addition salts, wherein the salts comprise the salts formed with the acids selected from the group consisting of hydrochloric acid,

methanesulfonic acid, sulfuric acid, phosphoric acid, citric acid, acetic acid and trifluoroacetic acid, preferably, the acids are hydrochloric acid and trifluoroacetic acid.

This invention relates to the preparation process of the compounds of formula (I), wherein the preparation process comprises the following steps of:

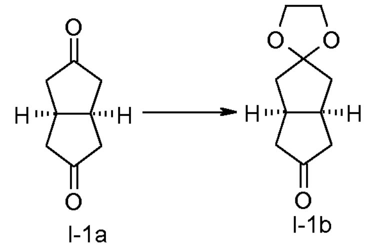

I-1a I-1b

reacting starting material tetrahydro-pentalene-2,5-dione(I-1a) with ethylene glycol and a catalyst of p-toluenesulfonic acid through refluxing in the solvent of benzene to give 7,7-(ethylidene acetal)bicyclo[3.3.0] octane-3-one protected (I-1b);

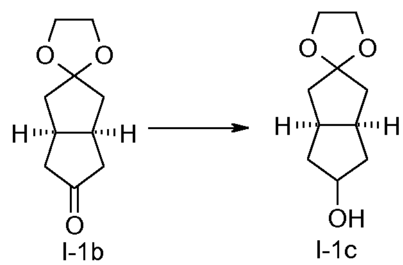

I-1b I-1c

reducing the obtained 7,7-(ethylidene acetal)bicyclo[3.3.0] octane-3-one (I-1b) by $NaBH_4$ at room temperature to afford 3-hydroxy-7,7-(ethylidene acetal)bicyclo[3.3.0] octane (1-1c);

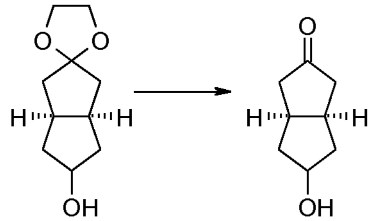

I-1c I-1e

reacting the obtained 3-hydroxy-7,7-(ethylidene acetal)bicyclo[3.3.0] octane (I-1c) with oxalic acid in the mixed solvent of ethyl acetate and water to give 5-hydroxy-hexahydro-pentalen-2-one (I-1e);

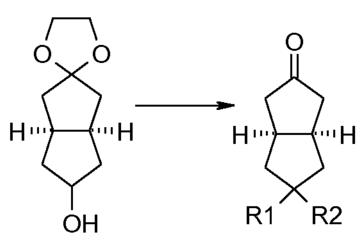

I-1c I-1d

or reacting the obtained 3-hydroxy-7,7-(ethylidene acetal)bicyclo[3.3.0] octane (I-1c) with different isocyanate and trimethylchlorosilane at room temperature or with different Grignard reagent in the solvent of ether and then acidifying it further by 2N hydrochloric acid to give the compounds of the formula (I-1d);

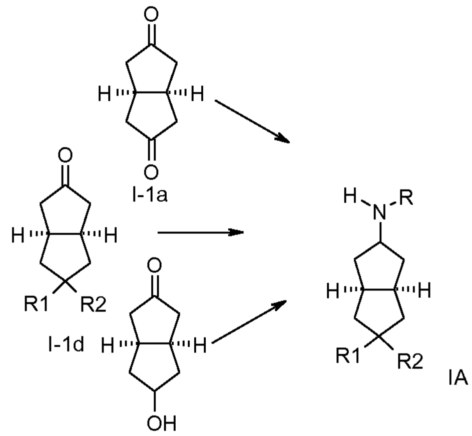

I-1a

I-1d

I-1c

IA

reacting the compounds of formula (I-1a) or formula (I-1d) or formula (I-1e) each independently with equivalent different amine in the solvent of methanol under the presence of sodium triethoxyborohydride and triethylamine at room temperature to give the compounds of formula (IA); wherein:

R is selected from the group consisting of alkyl, cycloalkyl, haloalkyl, aryl, heteroaryl, aminocarbonyl alkyl, amide alkyl, heterocyclo aminocarbonyl alkyl and aminoalkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;

$R_1$ and $R_2$ are each independently selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

wherein $R_1$ and $R_2$ are attached together with the atom to form a 3 to 8 membered ring, wherein the 3 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_6$ is selected from the group consisting of hydrogen or alkyl;

r is an integral from 1 to 6;

m is an integral from 0 to 6.

Preferably, in the above described preparation process, wherein R is heterocyclo aminocarbonyl alkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;

$R_1$ is hydrogen or hydroxyl; $R_2$ is selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

$R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_6$ is selected from the group consisting of hydrogen or alkyl;

r is an integral from 1 to 6;

m is an integral from 0 to 6.

Preferably, R is the formula:

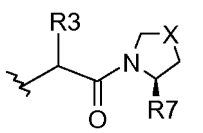

wherein, $R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;

$R_7$ is selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heteroalkyl, carboxylic acid, carboxylic ester and halogen;

X is C, S or O.

Further, the above described preparation process also comprises that the obtain compounds of formula (IA) through purification are directly reacted with acid in the solvent of ether under an ice-water bath to give the acid addition salt thereof, or reacted with di-*tert*-butyl dicarbonate to protect nitrogen atom, purified the compounds by silica gel column chromatography, then reacted with acid in the solvent of ether under an ice-water bath to give the acid addition salt thereof, wherein the acids are hydrochloric acid, methanesulfonic acid, sulfuric acid, phosphoric acid, citric acid, acetic acid and trifluoroacetic acid, preferably, the acids are hydrochloric acid and trifluoroacetic acid.

Further, in the above described preparation process, wherein the compounds of formula (I-1a) or formula (I-1d) or formula (I-1e) is each independently reacted with equivalent $RNH_2$ in the solvent of methanol under the presence of sodium triethoxyborohydride and triethylamine at room temperature to give the compounds of formula (IA); wherein

R is selected from the group consisting of alkyl, cycloalkyl, haloalkyl, aryl, heteroaryl, aminocarbonyl alkyl, amide alkyl, heterocyclo aminocarbonyl alkyl and aminoalkyl, wherein the heterocycle is selected from the group consisting of 5- or 6-membered hetero ring further substituted by one or more groups selected from the group consisting

of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen.

Preferably, R is the formula

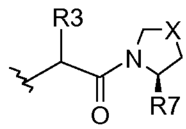

wherein:

$R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;

$R_7$ is selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heteroalkyl, carboxylic acid, carboxylic ester and halogen;

X is C, S or O.

In a particularly preferred embodiment, the compounds of formula (I) or pharmaceutically acceptable salts consisting of:

| Example No. | Structure | Name |
|---|---|---|
| 1 | 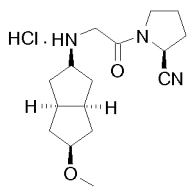 | 1-[2-(5-methoxy-octahydro-pentalen-2-ylamino)-acetyl]-pyrrolidine-2-carbonitrile hydrochloride |
| 2 | 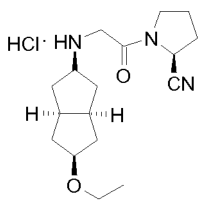 | 1-[2-(5-ethoxy-octahydro-pentalen-2-ylamino)-acetyl]-pyrrolidine-2-carbonitrile hydrochloride |
| 3 | 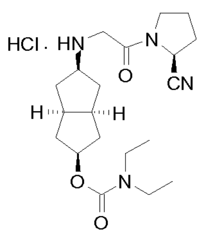 | 5-[2-(2-cyano-pyrrolidin-1-yl)-2-oxo-ethylamino]-octahydro-pentalen-2-yl diethyl-carbamate hydrochloride |

(continued)

| Example No. | Structure | Name |
|---|---|---|
| 4 | 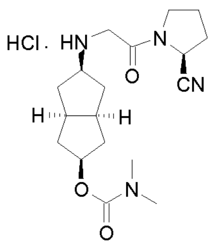 | 5-[2-(2-cyano-pyrrolidin-1-yl)-2-oxo -ethylamino]-octahydro-pentalen-2-yl dimethyl-carbamate hydrochloride |
| 5 | 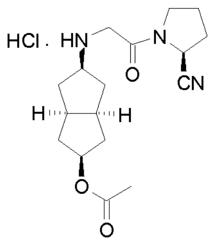 | 5-[2-(2-cyano-pyrrolidin-1-yl)-2-oxo-ethylamino]-octahydro-pentalen-2-yl acetate hydrochloride |
| 6 | 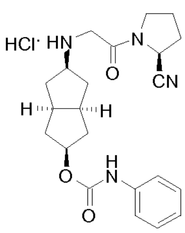 | 5-[2-(2-cyano-pyrrolidin-1-yl)-2-oxo-ethylamino]-octahydro-pentalen-2-yl phenylcarbamate hydrochloride |
| 7 | 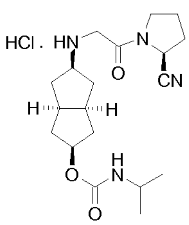 | 5-[2-(2-cyano-pyrrolidin-1-yl)-2-oxo -ethylamino]-octahydro-pentalen-2-yl isopropyl-carbamate hydrochloride |
| 8 | 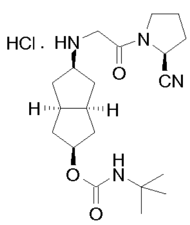 | 5-[2-(2-cyano-pyrrolidin-1-yl)-2-oxo-ethylamino]-octahydro-pentalen-2-yl tert-butyl-carbamate hydrochloride |
| 9 | 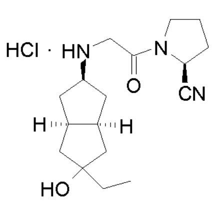 | 1-[2-(5-ethyl-5-hydroxy-octahydro-pentalen -2-ylamino)-1-hydroxy-ethyl]-pyrrolidine-2 -carbonitrile hydrochloride acid |

(continued)

| Example No. | Structure | Name |
|---|---|---|
| 10 | 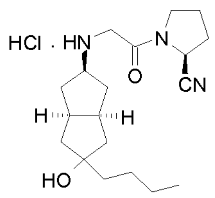 | 1-[2-(5-butyl-5-hydroxy-octahydro-pentalen-2-ylamino)-acetyl]-pyrrolidine-2-carbonitrile hydrochloride |
| 11 | 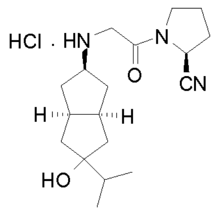 | 1-(2-(5-isopropyl-5-hydroxy-octahydro-pentalen-2-ylamino)acetyl) pyrrolidine-2-carbonitrile hydrochloride |
| 12 | 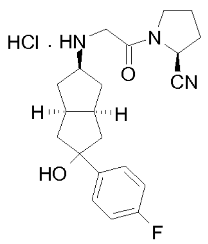 | 1- {2-[5-(4-fluoro-phenyl)-5-hydroxy-octahydro-pentalen-2-ylamino]-acetyl}-pyrrolidine-2-carbonitrile hydrochloride |
| 13 | 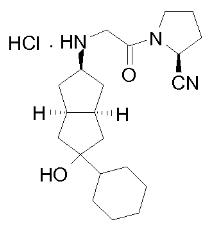 | 1-[2-(5-cyclohexyl-5-hydroxy-octahydro-pentalen-2-ylamino)-acetyl]-pyrrolidine -2-carbonitrile hydrochloride |
| 14 | 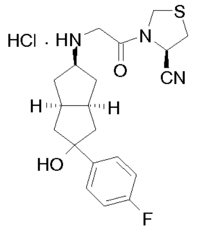 | 3-{2-[5-(4-fluoro-phenyl)-5-hydroxy-octahydro-pentalen-2-ylamino]-acetyl} -thiazolidine-2-carbonitrile hydrochloride |
| 15 | 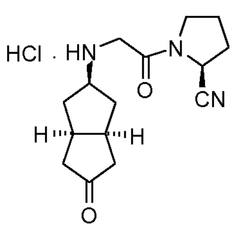 | 1-[2-(5-oxo-octahydro-pentalen-2-ylamino)-acetyl]-pyrrolidine-2-carbonitrile hydrochloride |

(continued)

| Example No. | Structure | Name |
|---|---|---|
| 16 | 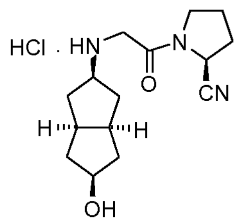 | 1-[2-(5-hydroxyl-octahydro-pentalen-2-ylamino)-acetyl]-pyrrolidine-2-carbonitrile hydrochloride |
| 17 | 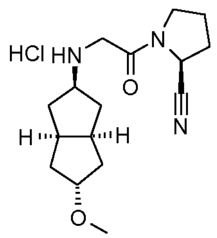 | 1-[2-(5-methoxy-octahydro-pentalen-2-ylamino)-acetyl]-pyrrolidine-2-carbonitrile hydrochloride |
| 18 | 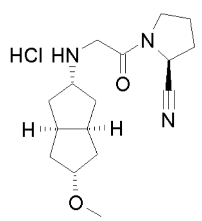 | 1-[2-(5-methoxy-octahydro-pentalen-2-ylamino)-acetyl]-pyrrolidine-2-carbonitrile hydrochloride |
| 19 | 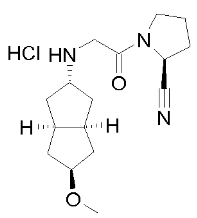 | 1-[2-(5-methoxy-octahydro-pentalen-2-ylamino)-acetyl]-pyrrolidine-2-carbonitrile hydrochloride |

Further, this invention relates to compounds as intermediates in the synthesis of compounds of formula (I) having the following formula (I-1d) or (I-1e):

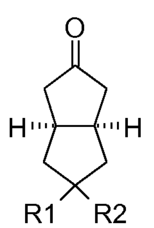

(I-1d)

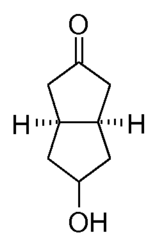

(I-1e)

wherein:

$R_1$ and $R_2$ are each independently selected from the group consisting of alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ or $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid

and carboxylic ester;
$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocycloalkoxyl, trifluoromethyl, carboxylic acid and carboxylic ester;
meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
r is an integral from 1 to 6;
m is an integral from 0 to 6.

Furthermore, this invention relates to the preparation process of compounds of formula (I-1d) or (I-1e), wherein the preparation process comprises the following steps of:

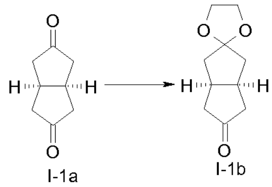

I-1a I-1b

reacting starting material tetrahydro-pentalene-2,5-dione (I-1a) with ethylene glycol and a catalyst of p-toluenesulfonic acid through refluxing in the solvent of benzene to give 7,7-(ethylidene acetal)bicyclo[3.3.0] octane-3-one protected (I-1b);

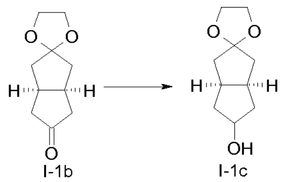

I-1b I-1c

reducing the obtained 7,7-(ethylidene acetal)bicyclo[3.3.0] octane-3-one (I-1b) by $NaBH_4$ at room temperature to afford 3-hydroxy-7,7-(ethylidene acetal)bicyclo[3.3.0] octane (I-1c);

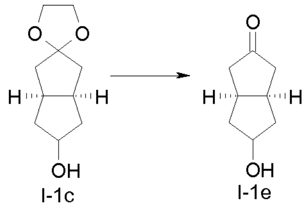

I-1c I-1e

reacting the obtained 3-hydroxy-7,7-(ethylidene acetal)bicyclo[3.3.0] octane (I-1c) with oxalic acid in the mixed solvent of ethyl acetate and water to give 5-hydroxy-hexahydro-pentalen-2-one (I-1e);

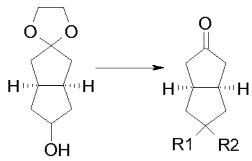

I-1c I-1d

or reacting the obtained 3-hydroxy-7,7-(ethylidene acetal)bicyclo[3.3.0] octane (I-1c) with different isocyanate and trimethylchlorosilane at room temperature or with different Grignard reagent in the solvent of ether and then acidifying it further by 2N hydrochloric acid to give the compounds of the formula (I-1d).

In another aspect, this invention relates to a pharmaceutical composition comprising compounds or salts thereof of formula (I) in an effective therapeutic dose, as well as pharmaceutically acceptable carrier.

In another aspect, this invention relates to a use of the compounds or salts of formula (I) in the preparation of a medicament as a dipeptidyl peptidase IV inhibitor (DPPIV).

In other words, this invention is intended to provide the new bicyclooctane derivatives of formula (IB) and their tautomers, enantiomers, non-enantiomers, raceme, and pharmaceutically acceptable salts, and metabolites and metabolic precursors or prodrugs.

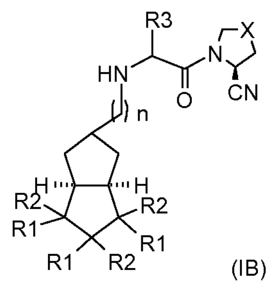

(IB)

wherein:

X is C, S or O;

$R_1$ and $R_2$ are each independently selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocy cloalkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

wherein $R_1$ and $R_2$ are attached together with the atom to form a 3 to 8 membered ring, wherein the 3 to 8 membered hetero ring further contains one or more heteroatoms selected from the group consisting of N, O and S, and the 3 to 8 membered hetero rings so formed are substituted by one ore more groups consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
n is an integral from 0 to 4;
r is an integral from 1 to 6;
m is an integral from 0 to 6.

Preferably, this invention relates to compounds or pharmaceutically acceptable salts of formula (IC):

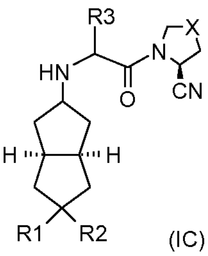
(IC)

X is C, S or O;
$R_1$ and $R_2$ are each independently selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$,- $NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;
wherein $R_1$ and $R_2$ are attached together with the atom to form a 3 to 8 membered ring, wherein the 3 to 8 membered hetero ring further contains one or more heteroatoms selected from the group consisting of N, O and S, and the 3 to 8 membered hetero rings so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;
$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;
meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered rings so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
r is an integral from 1 to 6;
m is an integral from 0 to 6.

This invention also relates to compounds as intermediates in the synthesis of compounds of formula (IB) having the following formula (I-1d) or (I-1e):

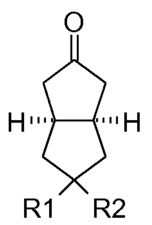

(I-1d)

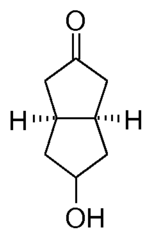

(I-1e)

wherein:

$R_1$ and $R_2$ are each independently selected from the group consisting of alkyl, cycloalkyl, heterocyclo alkyl, aryl, heteroaryl, $-OR_4$, $-(CH_2CH_2O)_rR_6$, $-(CH_2)_mC(O)OR_4$, $-(CH2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen or $-NR_4R_5$;

$R_6$ is selected from the group consisting of hydrogen or alkyl;

r is an integral from 1 to 6; and

m is an integral from 0 to 6.

This invention relates to compounds of formula (IB) or pharmaceutically acceptable salts, preferably:

$R_1$ is hydrogen or hydroxyl, $R_2$ is selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkylamino, amide group, alkoxyl, aryloxyl, heterocylco alkyl, carboxylic acid and carboxylic ester;

$R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;

$R_4$ and $R_5$ are independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid or carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_6$ is selected from the group consisting of hydrogen or alkyl;
r is an integral from 1 to 6; and
m is an integral from 0 to 6.

This invention relates to compounds of formula (IB) or pharmaceutically acceptable salts, preferably, X is C, S or O.

This invention relates to compounds of formula (IB) or pharmaceutically acceptable salts, preferably, n is 0.

A preparation process of compounds of formula (IC) or pharmaceutically acceptable salts, wherein the compounds of formula (I-1a), formula (I-1d) or formula (I-1e) are each independently reacted with different amine in the solvent of methanolunder the presence of sodium triethoxyborohydride and triethylamine at room temperature to give the compounds of formula (IC);

This invention relates to compounds of formula (IB) or pharmaceutically acceptable salts, wherein the compounds of formula (IB) are in pharmaceutically acceptable free-form and the forms of acid addition salts, and provide the pharmaceutically acceptable (nontoxic, physiologically acceptable) salts thereof; wherein the pharmaceutically acceptable salts are selected from the group consisting of hydrochloride, methanesulfonate, sulfate, phosphate, citrate, acetate and trifluoroacetate. Preferably, the salts are hydrochloride and trifluoroacetate. More preferably, the salts are hydrochloride.

## SYNTHESIS METHOD OF THE INVENTION COMPOUND

In order to complete the objection of the invention, the invention applies the following technical solution:

A preparation process of compounds of formula (IC) or pharmaceutically acceptable salts of the invention, comprising the following steps of:

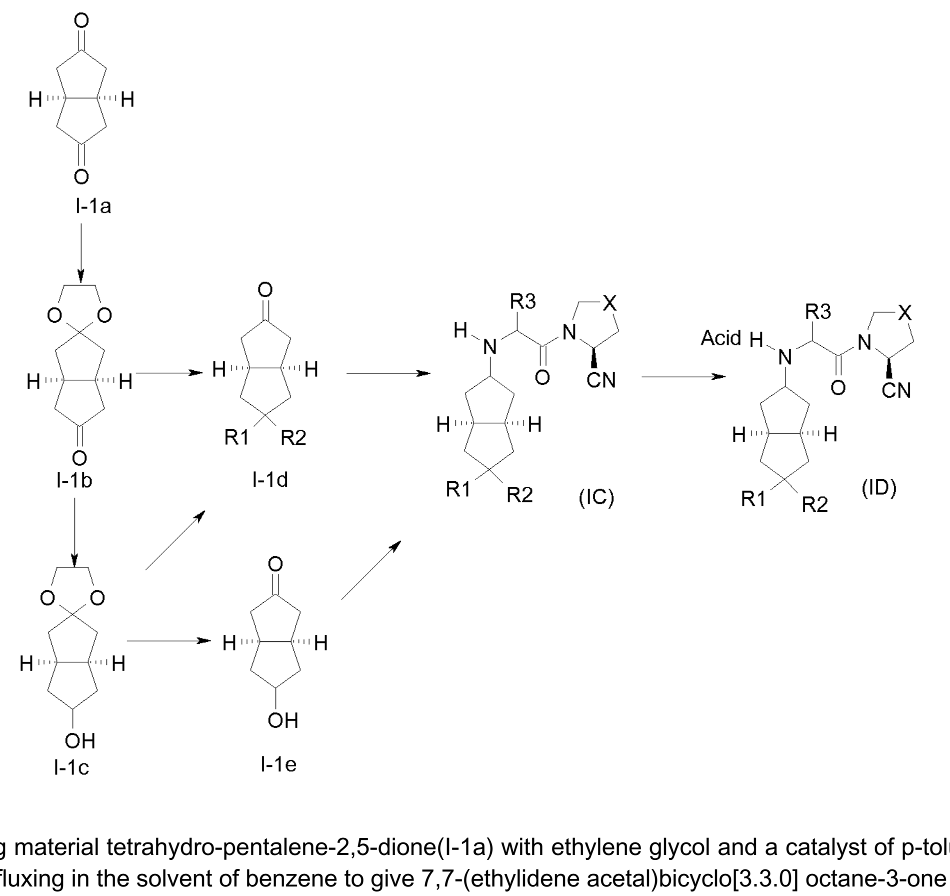


reacting starting material tetrahydro-pentalene-2,5-dione(I-1a) with ethylene glycol and a catalyst of p-toluenesulfonic acid through refluxing in the solvent of benzene to give 7,7-(ethylidene acetal)bicyclo[3.3.0] octane-3-one protected (I-1b); then reducing the obtained 7,7-(ethylidene acetal)bicyclo[3.3.0] octane-3-one (I-1b) by $NaBH_4$ at room temperature to afford 3-hydroxy-7,7-(ethylidene acetal)bicyclo[3.3.0] octane (I-1c); reacting the obtained 3-hydroxy-7,7-(ethylidene acetal)bicyclo[3.3.0] octane (I-1c) with oxalic acid in the mixed solvent of ethyl acetate and water to give 5-hydroxy-hexahydro-pentalen-2-one (I-1e); or reacting the obtained 3-hydroxy-7,7-(ethylidene acetal)bicyclo[3.3.0] octane (I-1c) with different isocyanate and trimethylchlorosilane at room temperature or with different Grignard reagent in the solvent

of ether and then acidifying it further by 2N hydrochloric acid to give the compounds of the formula (I-1d); reacting the compounds of formula (I-1a) or formula (I-1d) or formula (I-1e) each independently with equivalent different amine in the solvent of methanol under the presence of sodium triethoxyborohydride and triethylamine at room temperature to give the compounds of formula (IC); reacting the compounds of formula (IC) through purification directly with acid in the solvent of ether under an ice-water bath to give the acid addition salts thereof, or reacting the compounds with di-*tert*-butyl dicarbonate to protect nitrogen atom, purifying the compounds by silica gel column chromatography, then reacting the compounds with acid in the solvent of ether under an ice-water bath to give the acid addition salts thereof.

This invention relates to a pharmaceutical composition comprising a compound or salt in an effective therapeutic dose, as well as pharmaceutically acceptable carrier, or this invention relates to a use of the compounds or salts in the preparation of a medicament as a dipeptidyl peptidase IV inhibitor. In other words, this invention also provides the composition comprising the above compound in an effective therapeutic dose, and the use of the compounds in the preparation of a medicament as a dipeptidyl peptidase IV inhibitor.

## SPECIFIC IMPLEMENTION METHODS

The following examples serve to illustrate the invention, but the examples should not be considered as limiting the scope of the invention.

## EXAMPLES

The compound's structure determination was confirmed by NMR and MS. NMR chemical shifts were given in ppm ($10^{-6}$). NMR is determined by a Bruker AVANCE-400 machine. The solvent were deuterated-chloroform ($CDCl_3$) and deuterated-dimethyl sulfoxide (DMSO-*d6*) with tetramethylsilane (TMS) as internal standard. Chemical shifts were given in ppm ($10^{-6}$).

MS is determined by a FINNIGAN LCQ Ad (ESI) mass spectrometer.

$IC_{50}$ is determined by a NovoStar ELIASA (BMG Co. German).

Thin-layer silica gel is yantai huanghai HSGF254 or qingdao GF254 silica gel plate.

Column chromatography generally uses yantai huanghai 200-300 mesh silica gel as carrier.

DMSO-$D_6$: deuterated-dimethyl sulfoxide;

$CDCl_3$: deuterated-chloroform.

Example 1

Preparation of 1-[2-(5-methoxy-octahydro-pentalen-2-ylamino)-acetyl] -pyrrolidine-2-carbonitrile hydrochloride 1

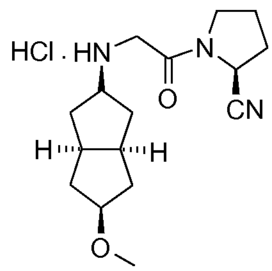

1

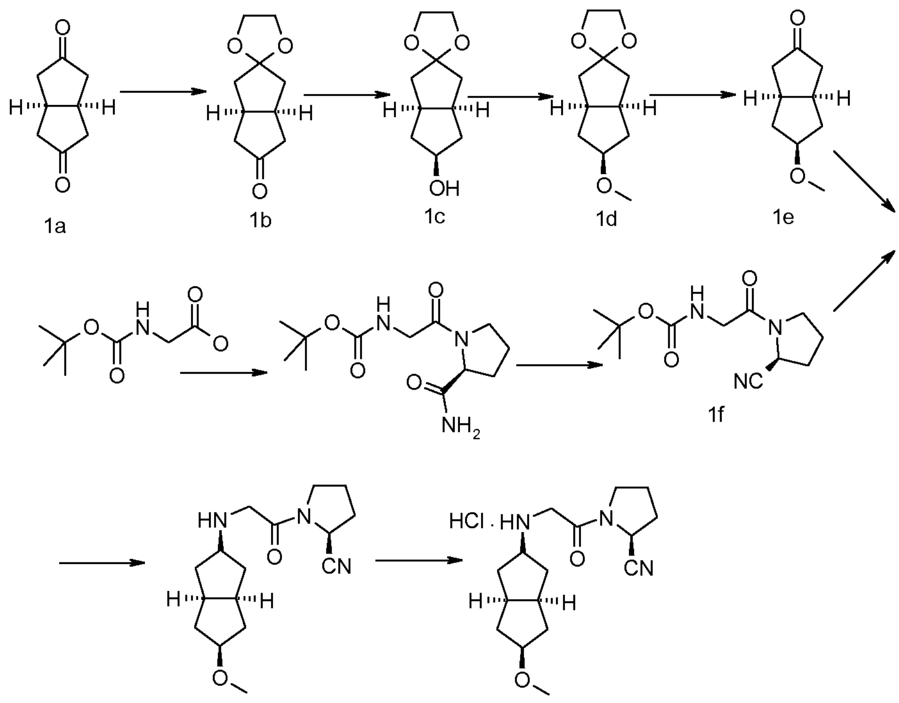

1a 1b 1c 1d 1e

1f

1g 1

Preparation of 7,7-(ethylidene acetal)bicyclo[3.3.0] octane-3-one 1b

In a 250ml round bottom flask equipped with a water segregator, tetrahydro-pentalene-2,5-dione **1a** (3 g, 21 mmol) and ethane-1,2-diol (1.03 mL, 18.5 mmol) were dissolved in 150 mL benzene under stirring, then 4-methylbenzenesulfonic acid (50 mg, 0.21 mmol) was added. Upon completion of the addition, the reaction mixture was heated to reflux overnight and then cooled to room temperature. The solvent of benzene was removed under reduced pressure. The residue was purified by silica gel column chromatography to give the title compound 7,7-(ethylidene acetal)bicyclo [3.3.0] octane-3-one **1b** (1.8 g, yield 56%) as a colorless oil.
MS m/z (ESI) : 183.5[M+1].

Preparation of 3-hydroxy-7,7-(ethylidene acetal)bicyclo[3.3.0] octane **1c**

7,7-(Ethylidene acetal)bicyclo[3.3.0] octane-3-one **1b** (1 g, 5.5 mmol) was dissolved in 15 mL methanol, sodium borohydride(0.4 g, 11 mmol) in batch was added in an ice-water bath. Upon completion of the addition, the reaction mixture was stirred at room temperature for 1-2 hours. The reaction was stoppted by adding acetone. The mixture was extracted with ethyl acetate (50 mL×3). The combined organic phase was washed with 50 mL saturated brine, dried over anhydrous sodium sulfate, filtered and concentrated under reduced pressure to give the title compound 3-hydroxy-7,7-(ethylidene acetal)bicyclo[3.3.0] octane **1c** (1.01 g, yield 99.9%) as a colorless oil.
MS m/z (ESI) : 185.2[M+1].

Preparation of 3-methoxy-7,7-(ethylidene acetal)bicyclo[3.3.0] octane **1d**

3-Hydroxy-7,7-(ethylidene acetal)bicyclo[3.3.0] octane **1c** (1 g, 5.4 mmol) was dissolved in 30 mL tetrahydrofuran, then sodium hydride(522 mg, 10.9 mmol) was added under stirring. Upon completion of the addition, the reaction mixture was stirred for 1 hour at room temperature, then added with methyl iodide (1.35 mL, 21.7 mmol) and heated to reflux for 3 hours. The reaction was stoppted by adding ice-water, the reaction mixture was extracted with ethyl acetate (30 mL×3). The combined organic phase was washed with 30 mL saturated brine, dried over anhydrous sodium sulfate, filtered and concentrated under reduced pressure. The residue was purified by silica gel column chromatography to give the title compound 3-methoxy-7,7-(ethylidene acetal)bicyclo[3.3.0] octane **1d** (944 mg, yield 88%) as a colorless oil.
MS m/z (ESI) : 199.6 [M+1].

Preparation of 5-methoxy- hexahydro-pentalen-2-one **1e**

3-Methoxy-7,7-(ethylidene acetal)bicyclo[3.3.0] octane **1d** (994mg, 5.02 mmol) was dissolved in the mixture of 30 mL ethyl acetate and 10 mL water, then ethanedioic acid(632 mg, 5.02 mmol) was added under stirring. Upon completion of the addition, the reaction mixture was stirred at room temperature until thin lay chromatography (TLC) showed the starting material disappeared, the reaction mixture was extracted with ethyl acetate(30 mL×3). The combined organic phase was washed with 30 mL saturated brine, dried over anhydrous sodium sulfate, filtered and concentrated under reduced pressure. The residue was purified by silica gel column chromatography to give the title compound 5-methoxy- hexahydro-pentalen-2-one **1e** (610 mg, yield 79%) as a light yellow oil.
MS m/z (ESI) : 155.1 [M+1].

Preparation of [2-(2-cyano-pyrrolidin-1-yl)-2-oxo-ethyl] -carbamic acid tert-butyl ester **1f**

1) Preparation of [2-(2-carbamoyl-pyrrolidin-1-yl)-2-oxo-ethyl]-carbamic acid tert-butyl ester

N-tert-butyloxycarbonyl glycine (5 g, 28.56 mmol) and L-pyrrolidine -2-carboxamide (3.25 g, 28.50 mmol) were dissolved in 75 mL N,N-dimethylformamide at 0 , then 1-hydroxybenzotriazole (11.8 g, 87.3 mmol) and N-ethyl-N'-(dimethylaminopropyl)-carbodiimide (11.3 g, 59 mmol) and triethylamine (12.1 mL, 87.3 mmol) were adding under stirring. Upon completion of the addition, the reaction mixture was naturally raised to room temperature, stirred overnight. The resulting mixture was concentrated under reduced pressure below 50 , extracted with ethyl acetate (200 mL×3). The combined organic phase was washed with 50 mL saturated brine, dried over anhydrous sodium sulfate, filtered and concentrated under reduced pressure. The residue was purified by recrystallization with ethyl acetate to give the title compound [2-(2-carbamoyl-pyrrolidin-1-yl)-2-oxo-ethyl]-carbamic acid tert-butyl ester 1f (7.42 g, yield 95.8%) as a white powder.
MS m/z (ESI) : 272.1[M+1].

2) Preparation of [2-(2-cyano-pyrrolidin-1-yl)-2-oxo-ethyl]-carbamic acid tert-butyl ester **1f**

In dry three-neck flask under a nitrogen atmosphere, 286 mL pyridine, [2-(2-carbamoyl-pyrrolidin-1-yl)-2-oxo-ethyl]-carbamic acid tert-butyl ester (13.5 g, 49.8 mmol) and imidazole (7.11 g, 104.6 mmol) were added in order, phosphorus oxychloride (19 mL, 204.2 mmol) was added at -35 under stirring. Upon completion of the addition, the reaction mixture was stirred for 1 hour at -35 , then naturally raised to room temperature and reacted for 0.5 hour. Pyridine was evaporated and the reaction mixture was extracted with ethyl acetate (200 mL×3). The combined organic phase was washed with 50 mL saturated brine, dried over anhydrous sodium sulfate, filtered and concentrated under reduced pressure. The residue was purified by silica gel column chromatography to give the title compound [2-(2-cyano-pyrrolidin-1-yl)-2-oxo-ethyl]-carbamic acid tert-butyl ester **1f** (10.7 g, yield 84.9%) as a white powder.
MS m/z (ESI) : 254.3[M+1].

Preparation of 1-[2-(5-methoxy-octahydro-pentalen-2-ylamino)-acetyl] pyrrolidine-2-carbonitrile **1g**

[2-(2-Cyano-pyrrolidin-1-yl)-2-oxo-ethyl]-carbamic acid tert-butyl ester **1f** (740 mg, 2.92 mmol) was dissolved in 20 mL dichloromethane, then trifluoroacetic acid (6.71 mL, 87.6 mmol) was added in an ice-water bath. Upon completion of the addition, the reaction mixture was stirred at 0 until the reaction was completed. Dichloromethane and trifluoroacetic acid were evaporated. The above residue was dissolved in 20 mL methanol, in order added with triethylamine (0.813 mL, 5.844 mmol), 5-methoxy-hexahydro-pentalen-2-one **1e** (310 mg, 1.948 mmol) and sodium triacetoxy borohydride (1.65 g, 7.792 mmol). Upon completion of the addition, the reaction mixture was stirred overnight at room temperature, concentrated under reduced pressure, added with 20 mL saturated sodium carbonate solution, extracted with dichloromethane (20 mL×3). The combined organic phase was washed with 10 mL saturated brine, the dichloromethane

phase was dried over anhydrous sodium sulfate, filtered and concentrated under reduced pressure. The residue was purified by silica gel column chromatography to give the title compound 1-[2-(5-methoxy-octahydro-pentalen-2-ylamino)-acetyl]pyrrolidine-2-carbonitrile **1g** (200 mg, yield 35.3%) as a white powder.
MS m/z (ESI) : 292.7[M+1].
$^1$H NMR ( 400 MHz, $CDCl_3$ ) δ( ppm ) 4.79(m, 1H), 3.82(m, 2H), 3.67(m, 2H), 3.48(m, 1H), 3.26(s, 3H), 2.40(m, 2H), 2.36-1.98(m, 6H), 1.85(m, 3H), 1.62(m, 2H), 1.53-1.14(m, 2H).

Preparation of 1-[2-(5-methoxy-octahydro-pentalen-2-ylamino)-acetyl] pyrrolidine-2-carbonitrile hydrochloride **1**

1-[2-(5-Methoxy-octahydro-pentalen-2-ylamino)-acetyl]pyrrolidine-2-carbonitril e **1g** (200 mg, 0.687 mmol) was dispersed in 10 mL ether, a solution of 0.5N hydrochloric acid in 2 mL ether was added in an ice-water bath. The resulting solid was centrifuged to give the title compound 1-[2-(5-methoxy-octahydro-pentalen-2-ylamino)-acetyl]pyrrolidine-2-carbonitrile hydrochloride **1** (180 mg, yield 80%) as a white powder.
MS m/z (ESI) : 292.7[M+1].

Example 2

Preparation of 1-[2-(5-ethoxy-octahydro-pentalen-2-ylamino)-acetyl] -pyrrolidine-2-carbonitrile hydrochloride 2

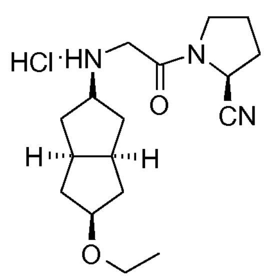

2

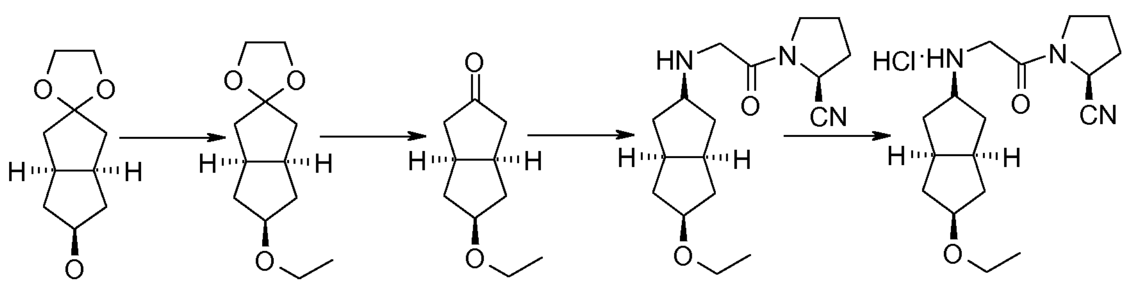

1c 2a 2b 2c 2

Preparation of 3-ethoxyl-7,7- (ethylidene acetal)bicyclo[3.3.0] octane **2a**

3-Hydroxy-7,7-(ethylidene acetal)bicyclo[3.3.0] octane **1c** (1 g, 5.4 mmol) was dissolved in 20 mL tetrahydrofuran, 50% sodium hydride (521 mg) was added under stirring. Upon completion of the addition, the reaction mixture was stirred for 1 hour, then added with ethyl iodide (0.869 mL, 10.86 mmol) and heated to reflux for 3 hours. The reaction was stopped by adding ice-water, the reaction mixture was extracted with ethyl acetate (80 mL×3). The combined organic phase was washed with 15 mL saturated brine, the ethyl acetate phase was dried over anhydrous sodium sulfate, filtered and concentrated under reduced pressure. The residue was purified by silica gel column chromatography to give the title compound 3-ethoxyl-7,7-(ethylidene acetal)bicyclo[3.3.0] octane **2a** (1.15 g, yield 100%) as a light yellow

oil.
MS m/z (ESI) : 213.2 [M+1].

Preparation of 5-ethoxy-hexahydro-pentalen-2-one **2b**

3-Ethoxyl-7,7-(ethylidene acetal)bicyclo[3.3.0] octane **2a** (1.15 g, 5.4 mmol) was dissolved in the mixture of 30 mL ethyl acetate and 10 mL water , then ethanedioic acid (1.368 g, 10.85 mmol) was added under stirring. Upon completion of the addition, the reaction mixture was stirred at room temperature until TLC showed the starting material **2a** disappeared, and the reaction mixture was extracted with ethyl acetate (50 mL×3). The combined organic phase was washed with 50 mL saturated brine, the ethyl acetate phase was dried over anhydrous sodium sulfate, filtered and concentrated under reduced pressure. The residue was purified by silica gel column chromatography to give the title compound 5-ethoxy-hexahydro-pentalen-2-one **2b** (871 mg, yield 80%) as a white powder.
MS m/z (ESI) : 169.3 [M+1].

Preparation of 1-[2-(5-ethoxy-octahydro-pentalen-2-ylamino)-acetyl] -pyrrolidine-2-carbonitrile **2c**

[2-(2-Cyano-pyrrolidin-1-yl)-2-oxo-ethyl]-carbamic acid tert-butyl ester **1f** (903 mg, 3.57 mmol) was dissolved in 20 mL dichloromethane in an ice-water bath, trifluoroacetic acid (8.2 mL, 107.1 mmol) was added. Upon completion of the addition, the reaction mixture was stirred at 0 until the reaction was completed. Dichloromethane and trifluoroacetic acid were evaporated. The above residue was dissolved in 20 mL methanol, in order added with triethylamine (0.993 mL, 7.143 mmol), 5-ethoxy-hexahydro-pentalen-2-one **2b** (400 mg, 2.38 mmol) and sodium triacetoxy borohydride (2.017 g, 9.52 mmol) at room temperature. The reaction mixture was concentrated and added with 50 mL saturated sodium carbonate solution, then extracted with dichloromethane (50 mL×3). The combined organic phase was washed with 50 mL saturated brine, the dichloromethane phase was dried over anhydrous sodium sulfate, filtered and concentrated under reduced pressure. The residue was purified by column chromatography to give the title compound 1-[2-(5-ethoxy-octahydro-pentalen-2-ylamino)-acetyl]-pyrrolidine-2-carbonitrile **2c** (182 mg, yield 30%) as a white powder.
MS m/z (ESI) : 306.5[M+1].
$^1$H NMR ( 400 $MH_z$, $CDCl_3$ ) δ( ppm ) 4.78(m, 1H), 3.89(m, 2H), 3.65(m, 2H), 3.44(m, 3H), 2.38(m, 2H), 2.32(m, 2H), 2.22(m, 2H), 2.13(m, 2H), 1.89-1.82(m, 2H), 1.58(m, 3H), 1.32-1.26(m, 2H) , 1.15(m, 3H).

Preparation of 1-[2-(5-ethoxy-octahydro-pentalen-2-ylamino)-acetyl] -pyrrolidine-2-carbonitrile hydrochloride **2**

1-[2-(5-Ethoxy-octahydro-pentalen-2-ylamino)-acetyl]-pyrrolidine-2-carbonitrile **2c** (182 mg, 0.598 mmol) was dispersed in 15 mL ether, a solution of 0.5N hydrochloric acid in 3 mL ether was added in an ice-water bath. The resulting solid was centrifuged to give the title compound 1-[2-(5-ethoxy-octahydro-pentalen-2 -ylamino)-acetyl]-pyrrolidine-2-carbonitrile hydrochloride **2** (160 mg, yield 80%) as a white powder.
MS m/z (ESI) : 306.4[M+1].

Example 3

Preparation of 5-[2-(2-cyano-pyrrolidin-1-yl)-2-oxo-ethylamino]-octahydro-pentalen-2-yl diethyl-carbamate hydrochloride 3

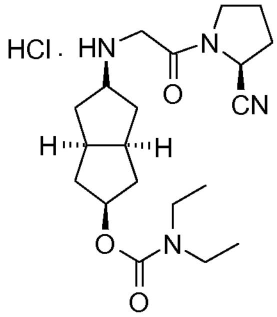

3

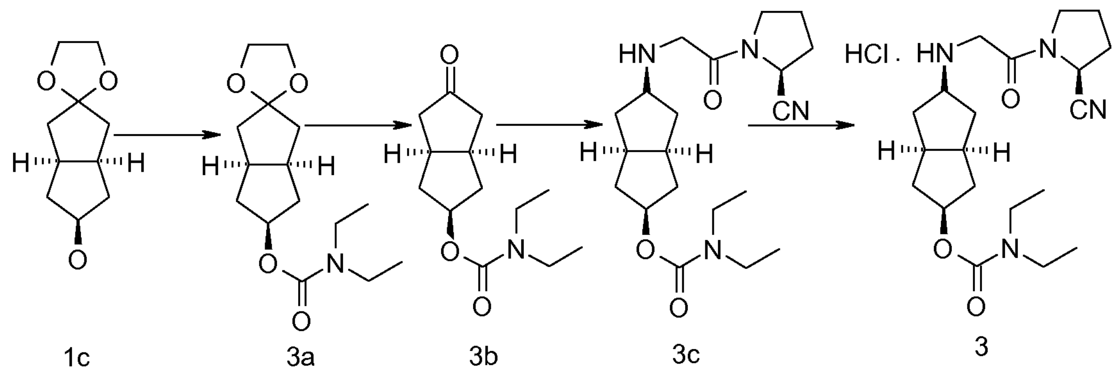

1c 3a 3b 3c 3

Preparation of 5,5-(ethylidene acetal)-octahydro-pentalen-2-yl diethyl-carbamate **3a**

Under an argon atmosphere, 3-hydroxy-7,7-(ethylidene acetal)bicyclo[3.3.0] octane **1c** (1.3 g, 7.065 mmol) was dissolved in 18 mL pyridine, then 4-dimethylamino pyridine (0.26 g, 2.12 mmol) was added, diethylamino formyl chloride (2.68 mL, 21.20 mmol) was added dropwise under stirring. Upon completion of the addition, the reaction mixture was heated to reflux overnight. Pyridine was evaporated after the reaction was completed, the reaction mixture was extracted with ethyl acetate (50 mL×3). The combined organic phase was washed with 50 mL saturated brine, the ethyl acetate was dried over anhydrous sodium sulfate, filtered and concentrated under reduced pressure. The residue was purified by silica gel column chromatography to give the title compound 5,5-(ethylidene acetal)-octahydro-pentalen-2-yl diethyl-carbamate **3a** (1.57 g, yield 88%) as a light yellow oil.
MS m/z (ESI) : 284.3 [M+1].

Preparation of 5-oxo-octahydro-pentalen-2-yl diethyl-carbamate **3b**

5,5-(Ethylidene acetal)-octahydro- pentalen-2-yl diethyl-carbamate **3a** (140 mg, 4.947 mmol) was dissolved in the mixture of 60 mL ethyl acetate and 15 mL water, then ethanedioic acid (623 mg, 4.947 mmol) was added under stirring. Upon completion of the addition, the reaction mixture was stirred at room temperature until TLC showed the starting material **3a** disappeared, and the reaction mixture was extracted with ethyl acetate (60 mL×3). The combined organic phase was washed with 50 mL saturated brine, the ethyl acetate phase was dried over anhydrous sodium sulfate, filtered and concentrated under reduced pressure. The residue was purified by silica gel column chromatography to give the title compound 5-oxo-octahydro-pentalen-2-yl diethyl-carbamate **3b** (1.02 g, yield 86%) as a white powder.
MS m/z (ESI) : 240.5 [M+1].

Preparation of 5-[2-(2-cyano-pyrrolidin-1-yl) -2-oxo-ethylamino]-octahydro-pentalen-2-yl diethyl-carbamate **3c**

[2-(2-Cyano-pyrrolidin-1-yl)-2-oxo-ethyl]-carbamic acid tert-butyl ester **1f** (530 mg, 2.1 mmol) was dissolved in 20 mL dichloromethane, then trifluoroacetic acid (4.8 mL, 63 mmol) was added under stirring in an ice-water bath. Upon completion of the addition, the reaction mixture was stirred at 0 until the reaction was completed. Dichloromethane and trifluoroacetic acid were evaporated. The above residue was dissolved in 20 mL methanol, in order added with triethylamine (0.88 mL, 6.3 mmol), 5-oxo-octahydro-pentalen-2-yl diethyl-carbamate **3b** (500 mg, 2.1 mmol) and sodium triacetoxy borohydride (1.34 g, 6.3 mmol). The reaction mixture was stirred overnight at room temperature, concentrated and added with 50 mL saturated sodium carbonate solution, extracted with dichloromethane (50 mL×3). The combined organic phase was washed with 50 mL saturated brine, the dichloromethane was dried over anhydrous sodium sulfate, filtered and concentrated under reduced pressure. The residue was purified by silica gel column chromatography to give the title compound 5-[2-(2-cyano-pyrrolidin-1-yl)-2-oxo-ethylamino]-octahydro-pentalen-2-yl diethyl-carbamate **3c** (400 mg, yield 50%) as a white powder.
MS m/z (ESI) : 377.2[M+1].
$^1$H NMR ( 400 MHz, $CDCl_3$ ) δ( ppm ) 5.07(m, 1H), 4.79(m, 1H), 3.92-3.40(m, 4H), 3.25(m, 4H), 2.39(m, 2H), 2.27(m, 2H), 2.20-2.12(m, 6H), 1.69(m, 1H), 1.57-1.54(m, 2H), 1.50-1.23(m, 2H), 1.11(m, 6H).

Preparation of 5-[2-(2-cyano-pyrrolidin-1-yl)-2-oxo-ethylamino]-octahydro -pentalen-2-yl diethyl-carbamate hydrochloride **3**

5-[2-(2-Cyano-pyrrolidin-1-yl)-2-oxo-ethylamino]-octahydro-pentalen-2-yl diethyl-carbamate **3c** (200 mg, 0.53 mmol) was dispersed in 15 mL ether, a 3 mL solution of 0.5N hydrochloric acid in ether was added in an ice-water bath. The resulting solid was centrifuged to give the title compound 5-[2-(2-cyano-pyrrolidin-1-yl)-2-oxo-ethylamino]-octahydro-pentalen-2-yl diethyl-carbamate hydrochloride **3** (180 mg, yield 82%) as a white powder.
MS m/z (ESI) : 377.3[M+1].

Example 4

Preparation of 5-[2-(2-cyano-pyrrolidin-1-yl)-2-oxo-ethylamino]-octahydro-pentalen-2-yl dimethyl-carbamate hydrochloride 4

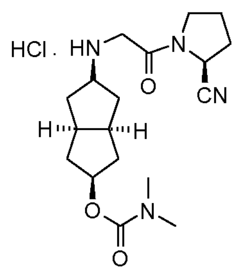

4

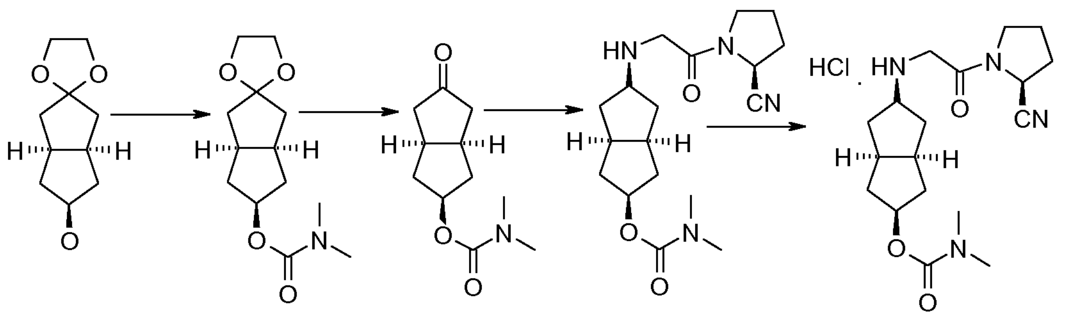

1c 4a 4b 4c 4

Preparation of 5,5-(ethylidene acetal)-octahydro-pentalen-2-yl dimethyl-carbamate **4a**

Under an argon atmosphere, 3-hydroxy-7,7-(ethylidene acetal)bicyclo[3.3.0]octane **1c** (1.3 g, 7.065 mmol) was dissolved in 15 mL pyridine, then 4-dimethylamino pyridine (0.26 g, 2.120 mmol) was added, dimethylamino formyl chloride (1.95 mL, 21.20 mmol) was added dropwise under stirring. Upon completion of the addition, the reaction mixture was heated to reflux overnight. Pyridine was evaporated after the reaction was completed, the reaction mixture was extracted with ethyl acetate (50 mL×3). The combined organic phase was washed with 15 mL saturated brine, the ethyl acetate phase was dried over anhydrous sodium sulfate, filtered and concentrated under reduced pressure. The residue was purified by silica gel column chromatography to give the title compound 5,5-(ethylidene acetal)-octahydro-pentalen-2-yl dimethyl-carbamate **4a** (1.8 g, yield 67%) as a light yellow oil.
MS m/z (ESI) : 256.0 [M+1].

Preparation of 5-oxo-octahydro-pentalen-2-yl dimethyl-carbamate **4b**

In an ice-water bath, dimethyl-carbamic acid 5,5-(ethylidene acetal)-octahydro-pentalen -2-yl ester **4a** (830 mg, 3.255 mmol) was dissolved in the mixture of ethyl acetate (60 mL) and water (10 mL), then ethanedioic acid (410 mg, 3.255 mmol) was added under stirring. Upon completion of the addition, the reaction mixture was stirred at room temperature until TLC showed the starting material **4a** disappeared, and the reaction mixture was extracted with ethyl

acetate (50 mL×3). The combined organic phase was washed with 15 mL saturated brine, the ethyl acetate phase was dried over anhydrous sodium sulfate, filtered and concentrated under reduced pressure. The residue was purified by silica gel column chromatography to give the title compound dimethyl-carbamic acid 5-oxo-octahydro-pentalen -2-yl ester **4b** (600 mg, yield 87%) as a light yellow oil.
MS m/z (ESI) : 212.2 [M+1].

Preparation of 5-[2-(2-cyano-pyrrolidin-1-yl)-2-oxo-ethylamino] -octahydro-pentalen-2-yl dimethyl-carbamate **4c**

[2-(2-Cyano-pyrrolidin-1-yl)-2-oxo-ethyl]-carbamic acid tert-butyl ester **1f** (835 mg, 3.3 mmol) was dissolved in 20 mL dichloromethane, then trifluoroacetic acid (7.66 mL, 100 mmol) was added under stirring in an ice-water bath. Upon completion of the addition, the reaction mixture was stirred at 0 until the reaction was completed. Dichloromethane and trifluoroacetic acid were evaporated. The above residue was dissolved in 20 mL methanol, in order added with triethylamine (0.922 mL, 6.6 mmol), dimethyl-carbamic acid 5-oxo-octahydro-pentalen-2-yl ester **4b** (460 mg, 2.2 mmol) and sodium triacetoxy borohydride (1.86 g, 8.8 mmol). The reaction mixture was stirred at room temperature, concentrated under reduced pressure, added with 20 mL saturated sodium carbonate solution, extracted with dichloromethane (60 mL×3). The combined organic phase was washed with 15 mL saturated brine, the dichloromethane was dried over anhydrous sodium sulfate, filtered and concentrated under reduced pressure. The residue was purified by silica gel column chromatography to give the title compound 5-[2-(2-cyano-pyrrolidin-1-yl) -2-oxo-ethylamino]-octahydro-pentalen-2-yl dimethyl-carbamate **4c** (400 mg, yield 52%) as a white powder.
MS m/z (ESI) : 349.4[M+1]o
$^1$H NMR ( 400 MHz, $CDCl_3$ ) δ( ppm) 5.06(m, 1H), 4.79(m, 1H), 4.01-3.37(m, 4H), 2.89(s, 6H), 2.42(m, 2H), 2.36-2.05 (m, 9H), 1.62(m, 2H), 1.54-1.26(m, 2H).

Preparation of 5-[2-(2-cyano-pyrrolidin-1-yl)-2-oxo-ethylamino]-octahydro -pentalen-2-yl dimethyl-carbamate hydrochloride **4**

5-[2-(2-Cyano-pyrrolidin-1-yl)-2-oxo-ethylamino]-octahydro-pentalen-2-yl dimethyl-carbamate **4c** (230 mg, 0.661 mmol) was dispersed in 15 mL ether, then a solution of 0.5N hydrochloric acid in 4 mL ether was added under stirring in an ice-water bath. The resulting solid was centrifuged to give the title compound 5-[2-(2-cyano-pyrrolidin-1-yl)-2-oxo-ethylamino]-octahydro-pentalen-2-yl dimethyl-carbamate hydrochloride **4** (200 mg, yield 78.7%) as a white powder.
MS m/z (ESI) : 349.2[M+1].

Example 5

Preparation of 5-[2-(2-cyano-pyrrolidin-1-yl)-2-oxo-ethylamino] -octahydro-pentalen-2-yl acetate hydrochloride 5

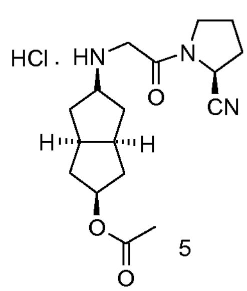

5

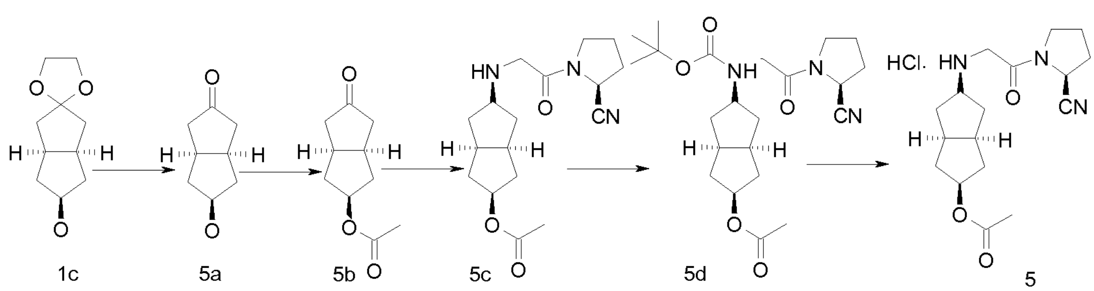

1c 5a 5b 5c 5d 5

Preparation of 5-hydroxy-hexahydro-pentalen-2-one **5a**

3-Hydroxy-7,7-(ethylidene acetal)bicyclo[3.3.0]octane **1c** (2.0 g, 10.87 mmol) was dissolved in the mixture of 60 mL ethyl acetate and 20 mL water , then ethanedioic acid (2.739 g, 21.74 mmol) was added. Upon completion of the addition, the reaction mixture was stirred overnight at room temperature until TLC showed the starting material **1c** disappeared, and the reaction mixture was extracted with ethyl acetate (60 mL×3). The combined organic phase was washed with 15 mL saturated brine, the ethyl acetate phase was dried over anhydrous sodium sulfate, filtered and concentrated under reduced pressure. The residue was purified by silica gel column chromatography to give the title compound 5-hydroxy-hexahydro-pentalen-2-one **5a** (1.12 g, yield 75%) as a light yellow oil.
MS m/z (ESI) : 141.3 [M+1].

Preparation of 5-oxo-octahydro-pentalen-2-yl acetate **5b**

In a dry three-neck flask, 5-hydroxy-hexahydro-pentalen-2-one **5a** (0.7 mg, 5 mmol) was dissolved in pyridine (15 mL), then 4-dimethylamino pyridine (0.18g, 1.5 mmol) was added, acetic anhydride (0.94 mL, 10 mL) was added dropwise under stirring. Upon completion of the addition, the reaction mixture was heated to reflux overnight. Pyridine was evaporated after the reaction was completed, the reaction mixture was extracted with ethyl acetate (50 mL×3). The combined organic phase was washed with 15 mL saturated brine, the ethyl acetate phase was dried over anhydrous sodium sulfate, filtered and concentrated under reduced pressure. The residue was purified by silica gel column chromatography to give the title compound 5-oxo-octahydro-pentalen-2-yl acetate **5b** (480 mg, yield 53.4%) as a light yellow oil.
MS m/z (ESI) : 183.6 [M+1].

Preparation of 5-[2-(2-cyano-pyrrolidin-1-yl)-2-oxo-ethylamino] -octahydro-pentalen-2-yl acetate **5c**

[2-(2-Cyano-pyrrolidin-1-yl)-2-oxo-ethyl]-carbamic acid tert-butyl ester **1f** (1.01 mg, 4.0 mmol) was dissolved in 20 mL dichloromethane and trifluoroacetic acid (9.2 mL, 120 mmol) in an ice-water bath. Upon completion of the addition, the reaction mixture was stirred at 0 until the reaction was completed. Dichloromethane and trifluoroacetic acid were evaporated. The above residue was dissolved in 20 mL methanol and in order added with triethylamine (1.12 mL, 8.0 mmol), acetic acid 5-oxo-octahydro-pentalen-2-yl ester **5b** (486 mg, 2.67 mmol) and sodium triacetoxy borohydride (1.97 g, 9.34 mmol). The reaction mixture was stirred at room temperature, concentrated and added with 20 mL saturated sodium carbonate solution, extracted with dichloromethane (80 mL×3). The combined organic phase was washed with 15 mL saturated brine, the dichloromethane phase was dried over anhydrous sodium sulfate, filtered and concentrated under reduced pressure. The residue was purified by silica gel column chromatography to give the title compound 5-[2-(2-cyano-pyrrolidin-1-yl)-2-oxo-ethylamino]-octahydro-pentalen-2-yl acetate **5c** (950 mg) as a white powder.
MS m/z (ESI) : 320.3[M+1].

Preparation of 5- {tert-butoxycarbonyl-[2-(2-cyano- pyrrolidin-1-yl)-2-oxo -ethyl]-amino}-octahydro-pentalen-2-yl acetate **5d**

5-[2-(2-Cyano-pyrrolidin-1-yl)-2-oxo-ethylamino]-octahydro-pentalen-2-yl acetate **5c** (crude product 950mg) was dissolved in dichloromethane (30 mL), potassium carbonate (1.38 g, 10 mmol), di-tert-butyl dicarbonate (1.08 g, 5 mmol) was added in order in an ice-water bath. Upon completion of the addition, the reaction mixture was stirred until TLC showed the starting material **5c** disappeared and added with water to stop the reaction, the reaction mixture was extracted with dichloromethane (80 mL×3). The combined organic phase was washed with 15 mL saturated brine, the dichloromethane was dired over anhydrous sodium sulfate, filtered and concentrated under reduced pressure. The residue was purified by silica gel column chromatography to give the title compound 5-{tert-butoxycarbonyl-[2-(2-cyano-pyrrolidin-1-yl)-2-oxo-ethyl]-amino}-octahydro-pentalen-2-yl acetate **5d** (350 mg, yield 36%) as a white powder.
MS m/z (ESI) : 320.4[M+1].
$^1$H NMR ( 400 MHz, $CDCl_3$ ) δ( ppm ) 5.16(m, 1H), 4.79(m, 1H), 3.98-3.37(m, 4H), 2.45(m, 2H), 2.36-1.94(m, 12H), 1.61 (m, 2H), 1.45(s, 9H), 1.54-1.24(m, 2H).

Preparation of 5-[2-(2-cyano-pyrrolidin-1-yl)-2-oxo-ethylamino] -octahydro-pentalen-2-yl acetate hydrochloride **5**

5-{Tert-butoxycarbonyl-[2-(2-cyano-pyrrolidin-1-yl)-2-oxo-ethyl]-amino}-octahydro-pentalen-2-yl acetate **5d** (350 mg, 0.835 mmol) was dispersed in 20 mL ether, a solution of 0.5N hydrochloric acid was added in 4 mL ether in an ice-water bath. After TLC showed the starting material **5d** disappeared, the resulting solid was separated by preparative chromatography to give the title compound 5-[2-(2-cyano-pyrrolidin-1-yl)-2-oxo-ethylamino]-octahydro-pentalen-2-yl acetate hydrochloride **5** (140 mg, yield 47%) as a white powder.
MS m/z (ESI) : 320.3[M+1].

Example 6

Preparation of 5-[2-(2-cyano-pyrrolidin-1-yl)-2-oxo-ethylamino]-octahydro-pentalen-2-yl phenyl-carbamate hydrochloride 6

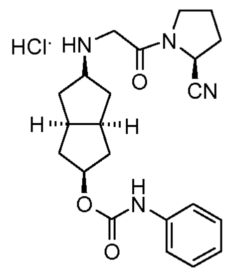

6

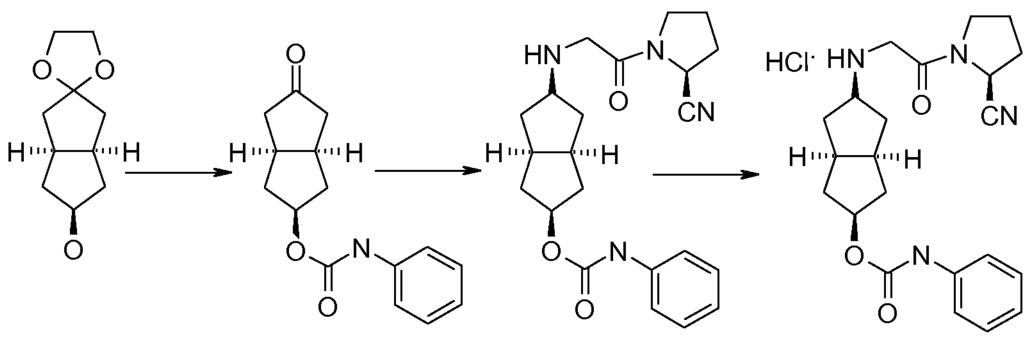

1c 6a 6b 6

Preparation of 5-oxo-octahydro-pentalen-2-yl phenyl-carbamate **6a**

Tricarbonyl chloride (2.97 g, 0.1mol) was dissolved in 20 mL toluene, a solution of amidobenzene (2.73 mL, 0.3 mol) in 10 mL toluene was added dropwise under stirring in an ice-water bath. Upon completion of the addition, the reaction mixture was stirred for 0.5 hour at room temperature, for another 2 hours at 50 , th$$en heated to reflux for 2 hours and cooled to room temperature. The reaction mixture was filtered to remove insoluble substance.
3-Hydroxy-7,7-(ethylidene acetal)bicyclo[3.3.0] octane **1c** (1.0 g, 5.4 mmol) was dissolved in 20 mL ethylene chloride in an ice-water bath, then the above isocyanate and trimethychlorosilicane (70 ul, 0.55 mmol)were added , then naturally raised to room temperature. The reaction mixture was stirred overnight at room temperature, added with water to stop the reaction, extracted with dichloromethane (30 mL×3). The combined organic phase was washed with 30 mL saturated brine, the dichloromethane phase was dried over anhydrous sodium sulfate, filtered and concentrated under reduced pressure.
The concentration was added with 20 mL ethyl acetate and 5 mL 2N hydrochloric acid, stirred for 1 hour at 35 and extracted with ethyl acetate (30 mL×3). The combined organic phase was washed with 30 mL saturated brine, the ethyl acetate was dried over anhydrous sodium sulfate, filtered and concentrated under reduced pressure. The residue was purified by silica gel column chromatography to give the title compound 5-oxo-octahydro-pentalen-2-yl phenyl-carbamate **6a** (1.0 g, yield 75%) as a white powder.
MS m/z (ESI) : 260.4[M+1].

Preparation of 5-[2-(2-cyano-pyrrolidin-1-yl)-2-oxo-ethylamino]-octahydro -pentalen-2-yl phenyl-carbamate **6b**

[2-(2-Cyano-pyrrolidin-1-yl)-2-oxo-ethyl]-carbamic acid tert-butyl ester **1f** (750 mg, 2.96 mmol) was dissolved in 20 mL dichloromethane, then trifluoroacetic acid (6.8 mL, 90 mmol) was added under stirring in an ice-water bath.

Upon completion of the addition, the reaction mixture was stirred at 0 until the reaction was completed. Dichloromethane and trifluoroacetic acid were evaporated. The above residue was dissolved in 20 mL methanol, in order added with triethylamine (1.03 mL, 7.1 mmol), 5-oxo-octahydro-pentalen-2-yl phenyl-carbamate **6a** (640 mg, 2.47 mmol) and sodium triacetoxy borohydride (2.1 g, 9.9 mmol). The reaction mixture was stirred at room temperature, concentrated and added with 20 mL saturated sodium carbonate solution, extracted with dichloromethane (80 mL×3). The combined organic phase was washed with 15 mL saturated brine, the dichloromethane was dried over anhydrous sodium sulfate, filtered and concentrated under reduced pressure. The residue was purified by silica gel column chromatography to give the title compound 5-[2-(2-cyano-pyrrolidin-1-yl)-2-oxo-ethylamino]-octahydro-pentalen-2-yl phenyl-carbamate **6b** (300 mg, yield 30.6%) as a white powder.
MS m/z (ESI) : 397.6[M+1].
$^1$H NMR ( 400 MHz, $CDCl_3$ ) δ( ppm ) 7.55(m, 1H), 7.27(m, 3H), 7.01(m, 1H), 5.32(m, 1H), 4.72(m, 1H), 4.32-3.93(m, 2H), 3.65(m, 2H), 2.55(m, 2H), 2.40-1.95(m, 7H), 1.94-1.60(m, 6H).

Preparation of 5-[2-(2-cyano-pyrrolidin-1-yl)-2-oxo-ethylamino]-octahydro-pentalen-2-yl phenyl-carbamate hydrochloride **6**

5-[2-(2-Cyano-pyrrolidin-1-yl)-2-oxo-ethylamino]-octahydro-pentalen-2-yl phenyl-carbamate **6b** (300 mg, 0.758 mmol) was dispersed in 15 mL ether, then a solution of 0.5N hydrochloric acid in 2 mL ether was added in an ice-water bath. The resulting solid was centrifuged to give the title compound 5-[2-(2-cyano-pyrrolidin-1-yl)-2-oxo-ethylamino]-octahydro-pentalen-2-yl phenyl-carbamate hydrochloride **6** (200 mg, yield 61 %) as a white powder.
MS m/z (ESI) : 397.3[M+1].

Example 7

Preparation of 5-[2-(2-cyano-pyrrolidin-1-yl)-2-oxo-ethylamino]-octahydro-pentalen-2-yl isopropyl-carbamate hydrochloride 7

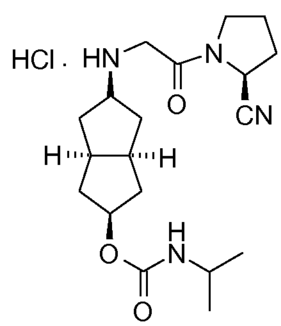

7

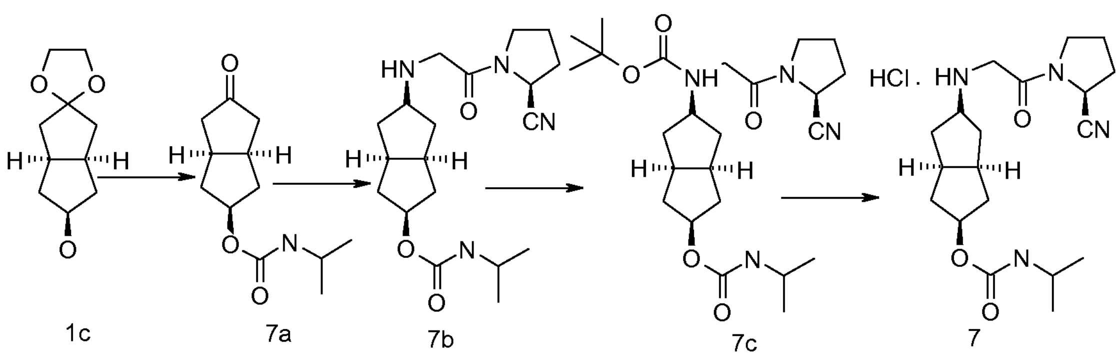

1c 7a 7b 7c 7

Preparation of 5-oxo-octahydro- pentalen-2-yl isopropyl-carbamate **7a**

Tricarbonyl chloride (2.97 g, 0.1mol) was dissolved in 20 mL toluene, then a solution of isopropylamine (2.6

mL, 30 mmol) in 10 mL toluene was added dropwise in an ice-water bath. Upon completion of the addition, the reaction mixture was stirred for 0.5 hour at room temperature, for another 2 hours at 50 , then heated to reflux for 2 hours and cooled to room temperature. The reaction mixture was filtered to remove insoluble substance.

3-Hydroxy-7,7-(ethylidene acetal)bicyclo[3.3.0] octane **1c** (1.0 g, 5.4 mmol) was dissolved in ethylene chloride (20 mL) in an ice-water bath, the above isocyanate and trimethychlorosilicane (70 ul, 0.55 mmol) were added, then naturally raised to room temperature. Upon completion of the addition, the reaction mixture was stirred overnight at room temperature and added with water to stop the reaction, the reaction mixture was extracted with dichloromethane (80 mL×3). The combined organic phase was washed with 15 mL saturated brine, the dichloromethane was dried over anhydrous sodium sulfate, filtered and concentrated under reduced pressure.

The concentration was added with 20 mL ethyl acetate and 5 mL 2N hydrochloric acid, stirred for 1 hour at 35 and extracted with ethyl acetate (80 mL×3). The combined organic phase was washed with 15 mL saturated brine, the ethyl phase was dried over anhydrous sodium sulfate, filtered and concentrated under reduced pressure. The residue was purified by silica gel column chromatography to give the title compound 5-oxo-octahydro- pentalen-2-yl isopropyl-carbamate **7a** (1.01 g, yield 81.8%) as a white powder.
MS m/z (ESI) : 226.2[M+1].

Preparation of 5-[2-(2-cyano-pyrrolidin-1-yl)-2-oxo-ethylamino]-octahydro -pentalen-2-yl isopropyl-carbamate **7b**

[2-(2-Cyano-pyrrolidin-1-yl)-2-oxo-ethyl]-carbamic acid tert-butyl ester **1f** (843 mg, 3.33 mmol) was dissolved in 30 mL dichloromethane, then trifluoroacetic acid (7.66 mL, 100 mmol) was added in an ice-water bath. Upon completion of the addition, the reaction mixture was stirred at 0 until the reaction was completed. Dichloromethane and trifluoroacetic acid were evaporated. The above residue was dissolved in 20 mL methanol and in order added with triethylamine (1.3 mL, 9.6 mmol), isopropyl-carbamic acid 5-oxo-octahydro-pentalen-2-yl ester **7a** (500 mg, 2.22 mmol) and sodium triacetoxy borohydride (2.2 g, 10 mmol). The reaction mixture was stirred at room temperature, concentrated under reduced pressure and added with 20 mL saturated sodium carbonate solution, then extracted with dichloromethane (60 mL×3). The combined organic phase was washed with 15 mL saturated brine, the dichloremethane phase was dried over anhydrous sodium sulfate, filtered and concentrated under reduced pressure to give the title compound 5-[2-(2-cyano-pyrrolidin-1-yl)-2-oxo-ethylamino]-octahydro-pentalen-2-yl isopropyl-carbamate **7b** (1.9 g) as a white powder, the crude product was directly used in the further reaction.
MS m/z (ESI) : 363.5[M+1].

Preparation of 5-{tert-butoxycarbonyl-[2-(2-cyano-pyrrolidin-1-yl)-2-oxo -ethyl]-amino}-octahydro-pentalen-2-yl isopropyl-carbamate **7c**

5-[2-(2-Cyano-pyrrolidin-1-yl)-2-oxo-ethylamino]-octahydro-pentalen-2-yl isopropyl-carbamate **7b** (1.9 g, 2.22 mmol) was dissolved in dichloromethane (30 mL), then potassium carbonate (1.53 g, 11.1 mmol), di-tert-butyl dicarbonate (1.21 g, 5.35 mmol) were added in order in an ice-water bath. Upon completion of the addition, the reaction mixture was stirred at room temperature until the starting material **7b** disappeared and added with water to stop the reaction, the reaction mixture was extracted with dichloromethane (60 mL×3). The combined organic phase was washed with 15 mL saturated brine, the dichloromethane was dired over anhydrous sodium sulfate, filtered and concentrated under reduced pressure. The residue was purified by silica gel column chromatography to give the title compound 5-{tert-butoxycarbonyl-[2-(2-cyano-pyrrolidin-1-yl)-2-oxo-ethyl]-amino}-octahydro-pentalen-2-yl isopropyl-carbamate **7c** (630 mg, yield 61.2%) as a white powder.
MS m/z (ESI) : 463.3[M+1].
$^1$H NMR ( 400 MHz, $CDCl_3$) δ( ppm ) 5.15(m, 1H), 4.76(m, 1H), 4.01(m, 2H), 3.81(m, 2H), 3.65(m, 2H), 2.45(m, 2H), 2.38-2.00(m, 6H), 1.98-1.60(m, 6H), 1.46(s, 9H), 1.18(m, 6H).

Preparation of 5-[2-(2-cyano-pyrrolidin-1-yl)-2-oxo-ethylamino]-octahydro -pentalen-2-yl isopropyl-carbamate hydrochloride 7

In a dry three-neck flask, 30 ml dichloromethane was added, 5-{tert-butoxycarbonyl-[2-(2-cyano-pyrrolidin-1-yl)-2-oxo-ethyl]-amino}-octahydro-pentalen-2-yl isopropyl-carbamate **7c** (360 mg, 0.779 mmol) was dispersed in 15 mL ether, then a solution of 0.5N hydrochloric acid in 3 mL ether was added in an ice-water bath. After the staring material **7c** disappeared, the solvent was evaporated under reduce pressure. The residue was seperated by column chromatography to give the title compound 5-[2-(2-cyano-pyrrolidin-1-yl)-2-oxo-ethylamino]-octahydro-pentalen-2-yl isopropyl-carbamate hydrochloride **7** (150 mg, yield 66%) as a white powder.
MS m/z (ESI) : 363.1[M+1].

Example 8

Preparation of 5-[2-(2-cyano-pyrrolidin-1-yl)-2-oxo-ethylamino]-octahydro-pentalen-2-yl tert-butyl-carbamate hydrochloride 8

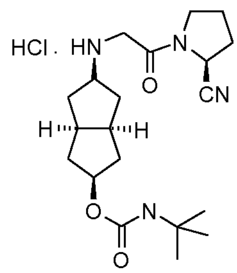

8

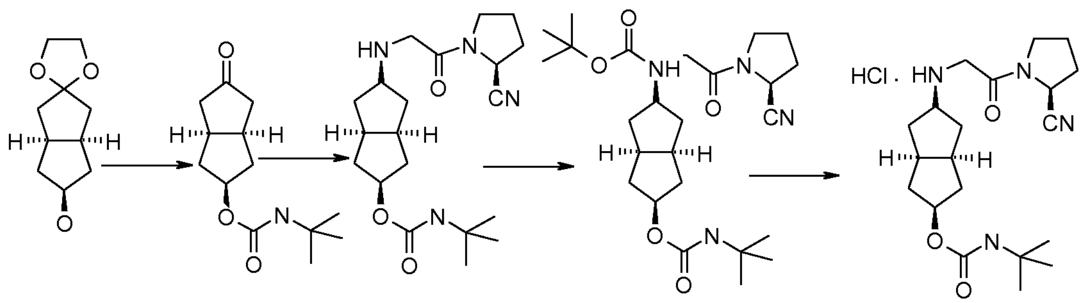

1c 8a 8b 8c 8

Preparation of 5-oxo-octahydro-pentalen-2-yl tert-butyl-carbamate **8a**

Tricarbonyl chloride (2.97 g, 0.1mol) was dissolved in toluene (20 mL) , then a solution of tert-butylamine (3.16 mL, 30 mmol) in toluene (10 mL) was added dropwise in an ice-water bath. Upon completion of the addition, the reaction mixture was stirred for 0.5 hour at room temperature, for another 2 hours at 50 , then heated to reflux for 2 hours and cooled to room temperature. The reaction mixture was filtered to remove insoluble substance.

3-Hydroxy-7,7-(ethylidene acetal)bicyclo[3.3.0] octane **1c** (1.0 g, 5.4 mmol) was dissolved in ethylene chloride (20 mL), and the above isocyanate and trimethychlorosilicane (70 ul, 0.55 mmol) was added in an ice-water bath, then naturally raised to room temperature. Upon completion of the addition, the reaction mixture was stirred overnight at room temperature and added with water to stop the reaction, the reaction mixture was extracted with dichloromethane (80 mL×3). The combined organic phase was washed with 15 mL saturated brine, the dichloromethane was dried over anhydrous sodium sulfate, filtered and concentrated under reduced pressure.

The concentration was added with 10 mL ethyl acetate and 5 mL 2N hydrochloric acid, stirred for 1 hour at 35 and extracted with ethyl acetate (60 mL×3). The combined organic phase was washed with 15 mL saturated brine, the ethyl acetate was dried over anhydrous sodium sulfate, filtered and concentrated under reduced pressure. The residue was purified by silica gel column chromatography to give the title compound 5-oxo-octahydro-pentalen-2-yl tert-butyl-carbamate **8a** (1.03 g, yield 79.4%) as a white powder.
MS m/z (ESI) : 240.2[M+1].

Preparation of 5-[2-(2-cyano-pyrrolidin-1-yl)-2-oxo-ethylamino]-octahydro-pentalen-2-yl tert-butyl-carbamate **8b**

[2-(2-Cyano-pyrrolidin-1-yl)-2-oxo-ethyl]-carbamic acid tert-butyl ester **1f** (794 mg, 3.318 mmol) was dissolved in 30 mL dichloromethane, then trifluoroacetic acid (7.2 mL, 94.18 mmol) was added in an ice-water bath. Upon completion of the addition, the reaction mixture was stirred at 0 until the reaction was completed. Dichloromethane and trifluoroacetic acid were evaporated. The above residue was dissolved in 20 mL methanol and in order added with triethylamine (0.87

mL, 6.276 mmol), 5-oxo-octahydro-pentalen-2-yl tert-Butyl-carbamate **8a** (500 mg, 2.092 mmol) and sodium triacetoxy borohydride (1.744 g, 8.368 mmol). The reaction mixture was stirred at room temperature, concentrated under the reduced pressure and added with 20 mL saturated sodium carbonate solution, extracted with dichloromethane (80 mL×3). The combined organic phase was washed with 15 mL saturated brine, the dichloromethane phase was dried over anhydrous sodium sulfate, filtered and concentrated under reduced pressure to give the title compound 5-[2-(2-cyano-pyrrolidin-1-yl)-2-oxo-ethylamino]-octahydro-pentalen-2-yl tert-butyl-carbamate **8b** (1.5 g) as a white powder, the crude product was directly used in the further reaction.
MS m/z (ESI) : 377.3[M+1].

Preparation of 5-{tert-butoxycarbonyl-[2-(2-cyano-pyrrolidin-1-yl)-2-oxo-ethyl] -amino}-octahydro-pentalen-2-yl tert-butyl-carbamate **8c**

5-[2-(2-Cyano-pyrrolidin-1-yl)-2-oxo-ethylamino]-octahydro-pentalen-2-yl tert-butyl-carbamate **8b** (1.5 g, 2.09 mmol) was dissolved in 30 mL dichloromethane, potassium carbonate (1.44 g, 10.46 mmol), di-tert-butyl dicarbonate (1.14 g, 5.23 mmol) were added in order in an ice-water bath. Upon completion of the addition, the reaction mixture was stirred until the starting material **8b** disappeared, then added with water to stop the reaction, the reaction mixture was extracted with dichloromethane (60 mL×3). The combined organic phase was washed with 15 mL saturated brine, the dichloromethane was dired over anhydrous sodium sulfate, filtered and concentrated under reduced pressure. The residue was purified by silica gel column chromatography to give the title compound 5-{tert-butoxycarbonyl-[2-(2-cyano-pyrrolidin-1-yl)-2-oxo-ethyl]-amino}-octahydro-pentalen-2-yl tert-butyl-carbamate **8c** (280 mg, yield 28.1 %) as a white powder.
MS m/z (ESI) : 477.1[M+1].

Preparation of 5-[2-(2-cyano-pyrrolidin-1-yl)-2-oxo-ethylamino]-octahydro-pentalen-2-yl tert-butyl-carbamate hydrochloride **8**

In a dry three-neck flask, 30 ml dichloromethane was added, 5-{tert-butoxycarbonyl-[2-(2-cyano-pyrrolidin-1-yl)-2-oxo-ethyl]-amino}-octahydro-pentalen-2-yl tert-butyl-carbamate **8c** (210 mg, 0.441 mmol) was dispersed in 15 mL ether, then a solution of 0.5N hydrochloric acid in 3 mL ether was added in an ice-water bath. After the staring material **8c** disappeared, the solvent was evaporated under reduce pressure. The residue was seperated by column chromatography to give the title compound 5-[2-(2-cyano-pyrrolidin-1-yl)-2-oxo-ethylamino]-octahydro- pentalen-2-yl tert-butyl-carbamate hydrochloride **8** (100 mg, yield 55%) as a white powder.
MS m/z (ESI) : 377.5[M+1].
$^1$H NMR ( 400 MHz, $CDCl_3$ ) δ( ppm ) 5.11(m, 1H), 4.76(m, 1H), 3.64(m, 2H), 2.44(m, 2H), 2.33-2.09(m, 8H), 2.06(m, 2H), 1.74-1.64(m, 5H), 1.47(s, 9H), 1.33(m, 9H).

Example 9

Preparation of 1-[2-(5-ethyl-5-hydroxy-octahydro-pentalen-2-ylamino) -1-hydroxy-ethyl]-pyrrolidine-2-carbonitrile hydrochloride 9

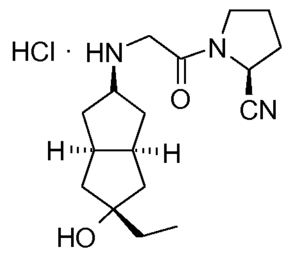

9

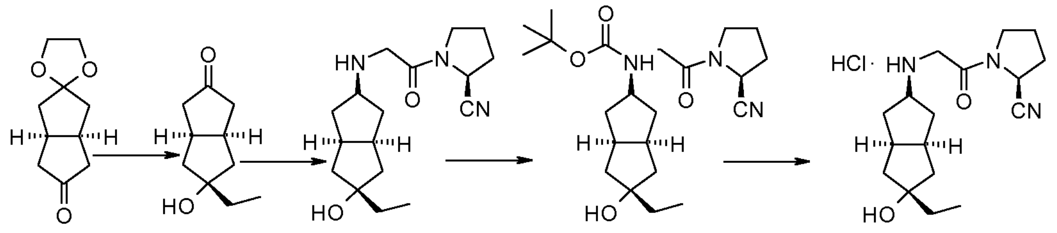

1c 9a 9b 9c 9

Preparation of 5-ethyl-5-hydroxy-hexahydro-pentalen-2-one **9a**

Magnesium (243 mg, 10 mmol) was added to 20 mL ether and lightly heated. The mixture was added with catalytic amount iodine and dropwise ethyl bromide (0.75 mL, 10 mmol) while maintaining the reaction system lightly boiled for 1 hour. The reaction mixture was dropwise added with a solution of 7,7- (ethylidene acetal)bicyclo[3.3.0] octane-3-one **1b** (0.8 g, 4.4 mmol) in 5 mL ether and then stirred for 2 hours, added with 10 mL 2N hydrochloric acid to stop the reaction for another 2 hours stirring, then the reaction mixture was extracted with ethyl acetate(30 mL×3). The combined organic phase was washed with 30 mL saturated brine, the ethyl acetate phase was dried over anhydrous sodium sulfate, filtered and concentrated under reduced pressure. The residue was purified by silica gel column chromatography to give the title compound 5-ethyl-5-hydroxy-hexahydro-pentalen-2-one **9a** (420 mg, yield 57%) as a light yellow oil.
MS m/z (ESI) : 169.2[M+1].

Preparation of 1-[2-(5-ethyl-5-hydroxy-octahydro-pentalen-2-ylamino)- acetyl]-pyrrolidine-2- carbonitrile **9b**

[2-(2-Cyano-pyrrolidin-1-yl)-2-oxo-ethyl]-carbamic acid tert-butyl ester **1f** (949 mg, 3.75 mmol) was dissolved in 30 mL dichloromethane, trifluoroacetic acid (8.62 mL, 112.5 mmol) was added in an ice-water bath. Upon completion of the addition, the reaction mixture was stirred at 0 until the reaction was completed. Dichloromethane and trifluoroacetic acid were evaporated. The above residue was dissolved in 20 mL methanol and in order added with triethylamine (1.04 mL, 7.5 mmol), 5-ethyl-5-hydroxy-hexahydro-pentalen-2-one **9a** (420 mg, 2.5 mmol) and sodium triacetoxy borohydride (2.385 g, 11.25 mmol). The reaction mixture was stirred o at room temperature, concentrated under reduced pressure and added with 20 mL saturated sodium carbonate solution, then extracted with dichloromethane (80 mL×3). The combined organic phase was washed with 15 mL saturated brine, the dichloromethane phase was dried over anhydrous sodium sulfate, filtered and concentrated under reduced pressure to give the title compound 1-[2-(5-ethyl-5-hydroxy-octahydro-pentalen-2-ylamino)-acetyl]-pyrrolidine-2-carbonitrile **9b** (700 mg) as a white powder, the crude product was directly used in the further reaction..
MS m/z (ESI) : 306.4[M+1].

Preparation of tert-butyl-[2-(2-cyano-pyrrolidin-1-yl)-2-oxo-ethyl]-(5-ethyl-5-hydroxy-octahydro-pentalen-2- yl)-carbamate **9c**

1-[2-(5-Ethyl-5-hydroxy-octahydro-pentalen-2-ylamino)-acetyl]-pyrrolidine-2-carbonitrile **9b** (700 mg, 2.5 mmol) was dissolved in 30 mL dichloromethane, then potassium carbonate (1.09 g, 6.25 mmol), di-tert-butyl dicarbonate (1.38 g, 12.5 mmol) were added in order in an ice-water bath. Upon completion of the addition, the reaction mixture was stirred at room temperature until the starting material **9b** disappeared, then added with water to stop the reaction, the reaction mixture was extracted with dichloromethane (80 mL×3). The combined organic phase was washed with 15 mL saturated brine, the dichloromethane phase was dried over anhydrous sodium sulfate, filtered and concentrated under

reduced pressure. The residue was purified by silica gel column chromatography to give the title compound tert-butyl-[2-(2-cyano-pyrrolidin-1-yl)-2-oxo-ethyl]-(5-ethyl-5-hydroxy-octahydro-pe ntalen-2- yl)-carbamate **9c** (355 mg, yield 35%) as a white powder.
MS m/z (ESI) : 406.2[M+1].
$^1$H NMR ( 400 MHz, $CDCl_3$ ) δ( ppm ) 4.78(m, 1H), 3.94-3.89(m, 3H), 2.48(m, 2H), 2.34-2.08(m, 6H), 1.75(m, 2H), 1.68-1.39(m, 17H), 0.93(m, 3H).

Preparation of 1-[2-(5-ethyl-5-hydroxy-octahydro-pentalen-2-ylamino) -1-hydroxy-ethyl]-pyrrolidine-2-carbonitrilehydrochloride **9**

In a dry three-neck flask, 10 ml dichloromethane was added, tert-butyl-[2-(2-cyano-pyrrolidin-1-yl)-2-oxo-ethyl]-(5-ethyl-5-hydroxy-octahydro-pentalen-2-yl)-car bamate **9c** (200 mg, 0.4938 mmol) was dispersed in 15 mL ether, then a solution of 0.5N hydrochloric acid in 3 mL ether was added in an ice-water bath. After the staring material **9c** disappeared, the solvent was evaporated under reduce pressure. The residue was seperated by column chromatography to give the title compound 1-[2-(5-ethyl-5-hydroxy-octahydro-pentalen-2-ylamino)-1-hydroxy-ethyl]-pyrrolidine -2-carbonitrilehydrochloride **9** (100 mg, yield 59.5%) as a white powder.
MS m/z (ESI) : 306.5[M+1].

Example 10

Preparation of 1-[2-(5-butyl-5-hydroxy-octahydro-pentalen-2-ylamino) -acetyl]-pyrrolidine-2-carbonitrile hydrochloride 10

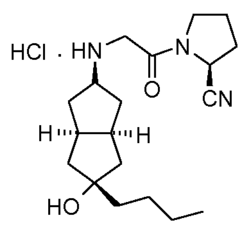

10

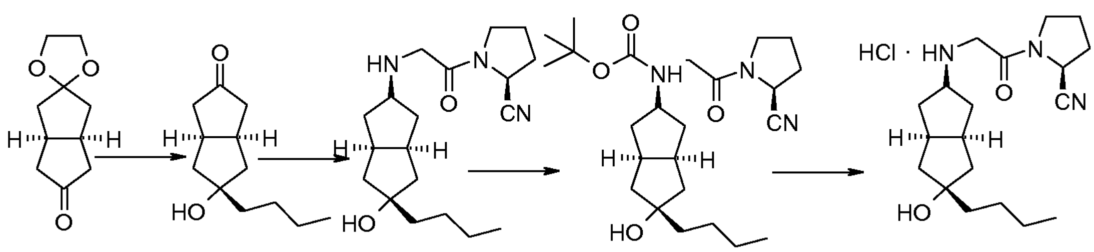

1b 10a 10b 10c 10

5-butyl-5-hydroxy-tetrahydro-pentalen-2-one **10a**

In a dry three-neck flask, butyl-magnesium chloride (2N in tetrahydrofuran, 4.8 mL) was dissolved in tetrahydrofuran (15mL), then a solution of 7,7-(ethylidene acetal)bicyclo[3.3.0] octane-3-one **1b**(1.46 g, 8 mmol) in 10 mL tetrahydrofuran was added dropwise in an ice-water bath. Upon completion of the addition, the reaction mixture was stirred for 2 hours, then added with 2 mL 2N hydrochloric acid to stop the reaction for another 2 hours strrring, and extracted with ethyl acetate (80 mL×3). The combined organic phase was washed with saturated brine (15 mL×1), the ethyl acetate phase was dried over anhydrous sodium sulfate, filtered and concentrated under reduced pressure. The residue was purified by silica gel column chromatography to give the title compound 5-butyl-5-hydroxy-tetrahydro-pentalen-2-one **10a** (620 mg, yield 35%) as a light yellow oil.
MS m/z (ESI) : 197.2[M+1].

Preparation of 1-(2-(5-butyl-5-hydroxy-octahydro-pentalen-2-ylamino)-acetyl) -pyrrolidine-2-carbonitrile **10b**

[2-(2-Cyano-pyrrolidin-1-yl)-2-oxo-ethyl]-carbamic acid tert-butyl ester **1f** (1.20 g, 4.7 mmol) was dissolved in 20 mL dichloromethane, then trifluoroacetic acid (10.8 mL, 141 mmol) was added in an ice-water bath. Upon completion of the addition, the reaction mixture was stirred at 0 until reaction was completed. Dichloromethane and trifluoroacetic acid were evaporated. The above residue was dissolved in 20 mL methanol and in order added with triethylamine (1.32 mL, 9.5 mmol), 5-butyl-5-hydroxy-hexahydro-pentalen-2-one **10a** (620 mg, 3.16 mmol) and sodium triacetoxy borohydride (3.0 g, 14.2 mmol). The reaction mixture was stirred at room temperature, concentrated under reduced pressure, and added with 20 mL saturated sodium carbonate solution, then extracted with dichloromethane (80 mL×3). The combined organic phase was washed with 15 mL saturated brine, the dichloromathine phase was dried over anhydrous sodium sulfate, filtered and concentrated under reduced pressure to give 1-(2-(5-butyl-5-hydroxy-octahydro-pentalen-2-ylamino) -acetyl)-pyrrolidine-2-carbonitrile **10b** (1.0 g) as a light yellow oil, the crude product was directly used in the further reaction.
MS m/z (ESI) : 334.5[M+1].
$^1$H NMR ( 400 $MH_z$, $CD_3OD$ ) δ( ppm ) 4.61(m, 1H), 3.48(m, 1H), 3.34(m, 3H), 2.90(m, 1H), 2.32(m, 2H), 2.10(m, 2H), 1.98(m, 4H), 1.70(m, 2H), 1.43(m, 2H), 1.31(m, 4H), 1.17(m, 4H), 0.76(m, 3H).

Preparation of tert-butyl-(5-Butyl-5-hydroxy-octahydro-pentalen-2-yl)-[2-(2-cyano-pyrrolidin-1-yl)-2-oxo-ethyl]-carbamate **10c**

1-[2-(5-Butyl-5-hydroxy-octahydro-pentalen-2-ylamino)-acetyl] -pyrrolidine-2-carbonitrile **10b** (900 mg, 2.7 mmol) was dissolved in 20 mL dichloromethane, then potassium carbonate (1.38 g, 10 mmol), di-tert-butyl dicarbonate (1.08 g, 5 mmol) were added in order in an ice-water bath. Upon completion of the addition, the reaction mixture was stirred until the starting material **10b** disappeared, then added with water to stop the reaction, the reaction mixture was extracted with dichloromethane (80 mL×3). The combined organic phase was washed with 15 mL saturated brine, the dichloremethane phase was dired over anhydrous sodium sulfate, filtered and concentrated under reduced pressure. The residue was purified by silica gel column chromatography to give the title compound tert-butyl-(5-butyl-5-hydroxy-octahydro-pentalen-2-yl)-[2-(2-cyano-pyrrolidin-1-yl) -2-oxo-ethyl]-carbamate 10c (350 mg, yield 30%) as a white powder.
MS m/z (ESI) : 434.3[M+1].

Preparation of 1-[2-(5-butyl-5-hydroxy-octahydro-pentalen-2-ylamino)-acetyl]- pyrrolidine-2-carbonitrilehydrochloride **10**

In a dry three-neck flask, 10 ml dichloromethane was added, tert-butyl-(5-butyl-5-hydroxy-octahydro-pentalen-2-yl)-[2-(2-cyano-pyrrolidin-1-yl)-2 -oxo-ethyl]-carbamate **10c** (200 mg, 0.462 mmol) was disperse in 15 mL ether, then a solution of 0.5N hydrochloric acid in 3 mL ether was added in an ice-water bath. After the staring material **10c** disappeared, the solvent was evaporated under reduce pressure. The residue was seperated by column chromatography to give the title compound 1-[2-(5-butyl-5-hydroxy-octahydro-pentalen-2-ylamino)-acetyl] -pyrrolidine-2-carbonitrilehydrochloride **10** (75 mg, yield 44%) as a white powder.
MS m/z (ESI) : 334.2[M+1].

Example 11

Preparation of 1-[2-(5-isopropyl-5-hydroxy-octahydro-pentalen-2-ylamino) acetyl]pyrrolidine-2-carbonitrile hydrochloride 11

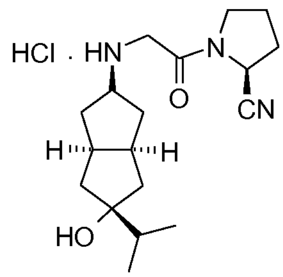

11

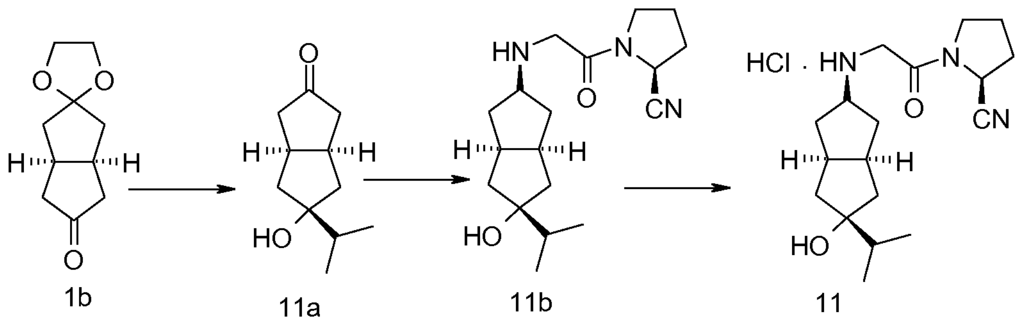

1b 11a 11b 11

Preparation of 5-hydroxy-5-isopropyl-hexahydro-pentalen-2-one **11a**

Magnesium (4.92 g, 40 mmol) was added to 10 mL ether and lightly heated. The mixture was stirred and added with catalytic amount iodine and dropwise 2-bromo propane (40 mL, 40 mmol) while maintaining the reaction system lightly boiled for 1 hour. The reaction mixture was dropwise added with a solution of 7,7-(ethylidene acetal)bicyclo[3.3.0] octane-3-one **1b** (1.82 g, 10 mmol) in 10 mL ether and then stirred for 2 hours, added with 2 mL 2N hydrochloric acid to stop the reaction for another 2 hours stirring and extracted with ethyl acetate (80 mL×3). The combined organic phase was washed with 15 mL saturated brine, the ethyl acetate phase was dried over anhydrous sodium sulfate, filtered and concentrated under reduced pressure. The residue was purified by silica gel column chromatography to give the title compound 5-hydroxy-5-isopropyl-hexahydro-pentalen-2-one **11a** (270 mg, yield 15%) as a light yellow oil.
MS m/z (ESI) : 282.2[M+1].

Preparation of 1-[2-(5-isopropyl-5-hydroxy-octahydro-pentalen-2-ylamino) -acetyl]pyrrolidine-2-carbonitrile **11b**

[2-(2-Cyano-pyrrolidin-1-yl)-2-oxo-ethyl]-carbamic acid tert-butyl ester **1f** (562 mg, 2.22 mmol) was dissolved in 20 mL dichloromethane, then trifluoroacetic acid (5.12 mL, 66.8 mmol) was added in an ice-water bath. Upon completion of the addition, the reaction mixture was stirred at 0 until reaction was completed. Dichloromethane and trifluoroacetic acid were evaporated. The above residue was dissolved in 20 mL methanol and in order added with triethylamine (0.62 mL, 4.44 mmol), 5-hydroxy-5-isopropyl-hexahydro-pentalen-2-one **11a**(270 mg, 1.48 mmol) and sodium triacetoxy borohydride (1.41 g, 6.66 mmol). The reaction mixture was stirred at room temperature, concentrated under reduce pressure and added with 20 mL saturated sodium carbonate solution, then extracted with dichloromethane (80 mL×3). The combined organic phase was washed with 15 mL saturated brine, the dichloromethane was dried over anhydrous sodium sulfate, filtered and concentrated under reduced pressure. The residue was purified by silica gel column chromatography to give the title compound 1-(2-(5-isopropyl-5-hydroxy -octahydro-pentalen-2-ylamino)-acetyl) pyrrolidine-2-carbonitrile **11b** (90 mg, yield 20%) as a white powder.
MS m/z (ESI) : 320.4[M+1].

Preparation of 1-[2-(5-isopropyl-5-hydroxy-octahydro-pentalen-2-ylamino)-acetyl]pyrrolidine-2-carbonitrile hydrochloride **11**

1-[2-(5-Isopropyl-5-hydroxy-octahydro-pentalen-2-ylamino)-acetyl]pyrrolidine -2-carbonitrile **11a** (90 mg, 0.282 mmol) was dispersed in 10 mL ether, then a solution of 0.5N hydrochloric acid in 2 mL ether was added in an ice-water bath. The resulting solid was centrifuged to give the title compound 1-(2-(5-isopropyl-5-hydroxy-octahydro-pentalen-2- ylamino)-acetyl)pyrrolidine-2-carbonitrile hydrochloride **11** (80 mg, yield 80%) as a light yellow powder.
MS m/z (ESI) : 320.5[M+1].

Example 12

Preparation of 1- {2-[5-(4-fluoro-phenyl)-5-hydroxy-octahydro-pentalen-2-ylamino] -acetyl}-pyrrolidine-2-carbonitrile hydrochloride 12

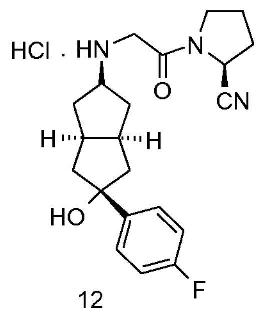

12

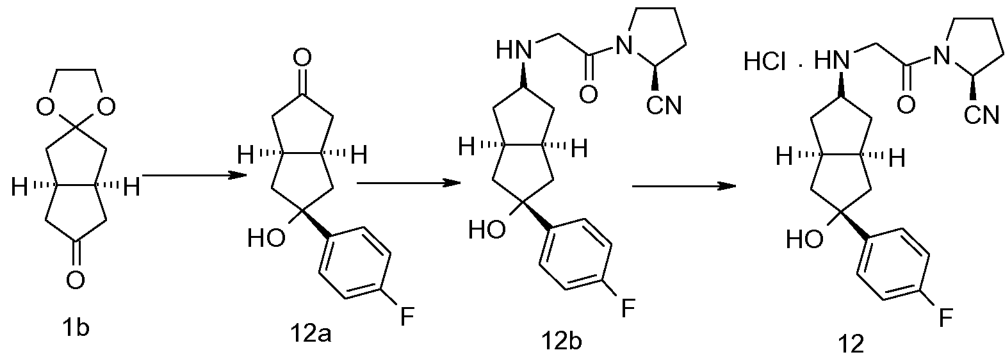

1b 12a 12b 12

Preparation of 5-(4-fluoro-phenyl)-5-hydroxy-hexahydro-pentalen-2-one **12a**

Magnesium (486 mg, 20 mmol) was added to 20 mL ether and lightly heated. The mixture was stirred and added with catalytic amount iodine and dropwise 1-bromo-4-fluoro-benzene (2.4 mL, 22 mmol) while maintaining the reaction system lightly boiled for 1 hour. The reaction mixture was dropwise added with a solution of 7,7-(ethylidene acetal)bicyclo[3.3.0] octane-3-one **1b** (1.19 g, 6.54 mmol) in 10 mL ether and then was stirred for 2 hours, added with 2 mL 2N hydrochloric acid to stop the reaction for another 2 hours stirring and extracted with ethyl acetate (80 mL×3). The combined organic phase was washed with 15 mL saturated brine, the ethyl acetate phase was dried over sodium sulfate, filtered and concentrated under reduced pressure. The residue was dissolved in the mixture of 30 mL dichloromethane, 10 mL water, ethanedioic acid (1.18g, 9.35mL) and then was stirred overnight. The reaction mixture was extracted with ethyl acetate (80 mL×3). The combined organic phase was washed with 15 mL saturated brine, the ethyl acetate phase was dried over anhydrous sodium sulfate, filtered and concentrated under reduced pressure. The residue was purified by silica gel column chromatography to give the title compound 5-(4-fluoro-phenyl)-5-hydroxy-hexahydro-pentalen-2-one **12a** (900 mg, yield 60%) as a white powder.
MS m/z (ESI) : 235.3[M+1].

Preparation of 1- {2-[5-(4-fluoro-phenyl)-5-hydroxy-octahydro-pentalen-2-ylamino]-acetyl}-pyrrolidine-2-carbonitrile **12b**

[2-(2-Cyano-pyrrolidin-1-yl)-2-oxo-ethyl]-carbamic acid tert-butyl ester **1f** (680 mg, 2.69 mmol) was dissolved in 30 mL then trifluoroacetic acid (6.18 mL, 80.7 mmol) was added in an ice-water bath. Upon completion of the addition, the reaction mixture was stirred at 0 until reaction was completed. Dichloromethane and trifluoroacetic acid were evaporated. The above residue was dissolved in 20 mL methanol and in order added with triethylamine (0.747 mL, 5.37 mmol), 5-(4-fluoro-phenyl) -5-hydroxy-hexahydro-pentalen-2-one **12a** (420 mg, 1.79 mmol) and sodium triacetoxy borohydride (1.71 g, 8.06 mmol). The reaction mixture was stirred at room temperature, concentrated under reduced pressure and added with 20 mL saturated sodium carbonate solution, then extracted with dichloromethane (80 mL×3). The combined organic phase was washed with 15 mL saturated brine, the dichloremethane phase was dried over anhydrous sodium sulfate, filtered and concentrated under reduced pressure. The residue was purified by silica gel column chromatography to give the title compound 1-(2-(5-(4-fluoro-phenyl)-5-hydroxy-octahydro-pentalen-2-ylamino)-acetyl)-pyrrolidine-2-carbonitrile **12b** (110 mg, yield 16.6%) as a white powder.
MS m/z (ESI) : 372.3[M+1].
$^1$H NMR ( 400 MHz, $CDCl_3$ ) δ( ppm) 7.44(m, 2H), 6.97(m, 2H), 4.75(m, 1H), 3.64-3.22(m, 5H), 2.86(m, 2H), 2.40(m, 2H), 2.36-2.04(m, 8H), 1.82(m, 2H).

Peparation of 1- {2-[5-(4-fluoro-phenyl)-5-hydroxy-octahydro-pentalen-2-ylamino] -acetyl}-pyrrolidine-2-carbonitrile hydrochloride **12**

1-{2-[5-(4-Fluoro-phenyl)-5-hydroxy-octahydro-pentalen-2-ylamino]-acetyl} -pyrrolidine-2-carbonitrile **12b** (110 mg, 0.296 mmol) was dispersed in 10 mL ether, then a solution of 0.5N hydrochloric acid in 2 mL ether was added in an ice-water bath. The resulting solid was centrifuged to give the title compound 1-{2-[5-(4-fluoro-phenyl)-5-hydroxy-octahydro-pentalen-2-ylamino]-acetyl)-pyrrolidine-2-carbonitrile hydrochloride **12** (100 mg, yield 83%) as a white powder.
MS m/z (ESI) : 372.4[M+1].

Example 13

Preparation of 1-[2-(5-cyclohexyl-5-hydroxy-octahydro-pentalen-2-ylamino) -acetyl]-pyrrolidine-2-carbonitrile hydrochloride 13

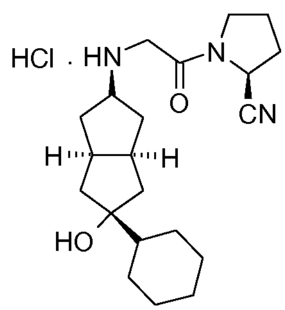

13

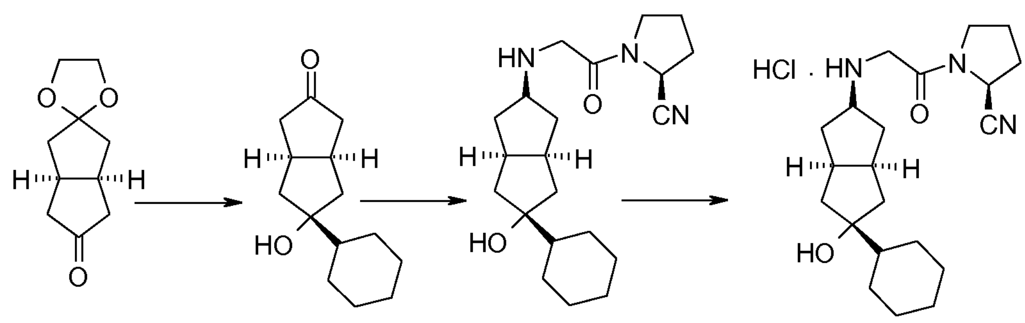

1b 13a 13b 13

Preparation of 5-cyclohexyl-5-hydroxy-hexahydro-pentalen-2-one **13a**

Magnesium (486 mg, 20 mmol) was added to 10 mL ether and lightly heated. The mixture was stirred and added with catalytic amount iodine and dropwise 1-chloro-hexane (2.48 mL, 21 mmol) while maintaining the reaction system lightly boiled for 1 hour. The reaction mixture was dropwise added with a solution of 7,7-(ethylidene acetal) bicyclo[3.3.0] octane-3-one **1b** (0.546 g, 3 mmol) in 10 mL ether and then was stirred for 2 hours, added with 2 mL 2N hydrochloric acid to stop the reaction for another 2 hours stirring and extracted with ethyl acetate (80 mLx3). The

combined organic phase was washed with 15 mL saturated brine, the ethyl acetate phase was dried over anhydrous sodium sulfate, filtered and concentrated under reduced pressure. The residue was dissolved in the mixture of 10 mL ethyl acetate, 5 mL water, ethanedioic acid (189 mg, 1.5 mmol) and then stirred for 6h at 40 . The reaction mixture was extracted with ethyl acetate (80 mLx3). The combined organic phase was washed with 15 mL saturated brine, the ethyl acetate phase was dried over anhydrous sodium sulfate, filtered and concentrated under reduced pressure. The residue was purified by silica gel column chromatography to give the title compound 5-cyclohexyl-5-hydroxy-hexahydro-pentalen-2-one **13a** (110 mg, yield 54%) as a light yellow powder.
MS m/z (ESI) : 223.1[M+1].

Preparation of 1-[2-(5-cyclohexyl-5-hydroxy-hexahydro-pentalen -2-ylamino)-acetyl]-pyrrolidine-2-carbonitrile **13b**

[2-(2-Cyano-pyrrolidin-1-yl)-2-oxo-ethyl]-carbamic acid tert-butyl ester **1f** (479 mg, 1.89 mmol) was dissolved in 20 mL dichloromethane, then trifluoroacetic acid (4.34 mL, 56.7 mmol) was added in an ice-water bath. Upon completion of the addition, the reaction mixture was stirred at 0 until reaction was completed. Dichloromethane and trifluoroacetic acid were evaporated. The above residue was dissolved in 20 mL methanol, in order added with triethylamine (0.66 mL, 4.74 mmol), 5-cyclohexyl-5-hydroxy -hexahydro-pentalen-2-one **13a** (350 mg, 1.58 mmol) and sodium triacetoxy borohydride (0.51 g, 7.11 mmol). The reaction mixture was stirred at room temperature, concentrated under reduced pressure, added with 20 mL saturated sodium carbonate solution, the extracted with dichloromethane (80 mLx3). The combined organic phase was washed with 15 mL saturated brine, the dichloromethane phase was dried over anhydrous sodium sulfate, filtered and concentrated under reduced pressure. The residue was purified by silica gel column chromatography to give the title compound 1-[2-(5-cyclohexyl-5-hydroxy -hexahydro-pentalen-2-ylamino]-acetyl)-pyrrolidine-2- carbonitrile **13b** (90 mg, yield 16.0%) as a white powder.
MS m/z (ESI) : 360.3[M+1].

Preparation of 1-[2-(5-cyclohexyl-5-hydroxy-octahydro-pentalen-2-ylamino) -acetyl]-pyrrolidine-2-carbonitrile hydrochloride **13**

1-[2-(5-Cyclohexyl-5-hydroxy-hexahydro-pentalen-2-ylamino)-acetyl]-pyrrolidine-2-carbonitrile **13b** (90 mg, 0.28 mmol) was dispersed in 10 mL ether, then a solution of 0.5N hydrochloric acid in 2 mL ether was in an ice-water bath. The rection mixture was evaporated to give the title compound 1-[2-(5-cyclohexyl-5-hydroxy-octahydro-pentalen-2-ylamino)-acetyl]-pyrrolidine-2-c arbonitrile hydrochloride **13** (80 mg, yield 80%) as a white powder.
MS m/z (ESI) : 360.2[M+1].

Example 14

Preparation of 3- {2-[5-(4-Fluoro-phenyl)-5-hydroxy-octahydro-pentalen-2-ylamino] -acetyl}-thiazolidine-2-carbonitrile hydrochloride 14

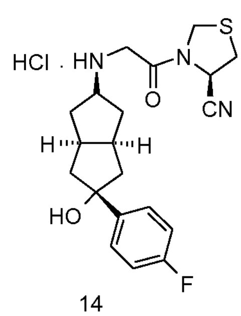

14

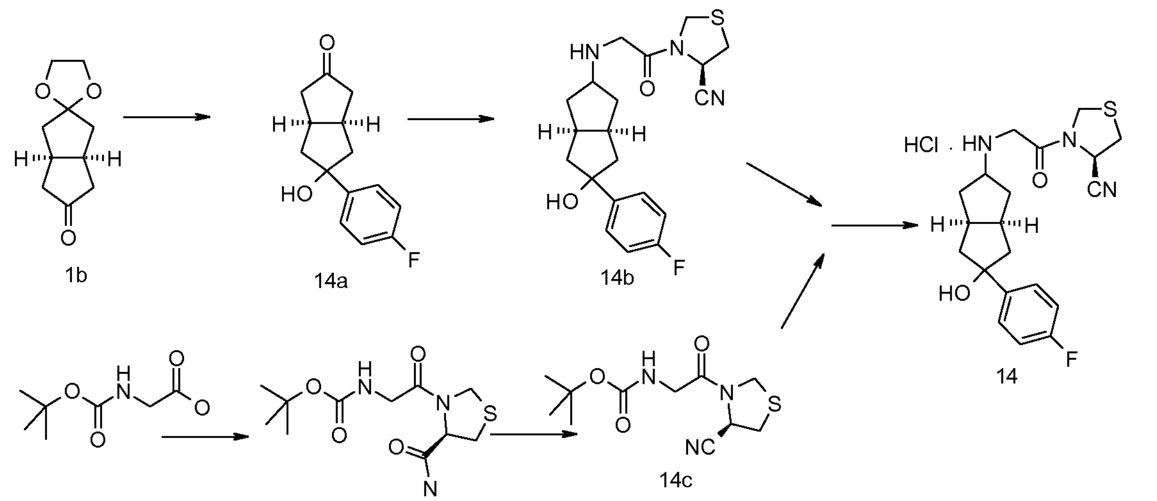

1b 14a 14b 14 14c

Preparation of 5-(4-fluoro-phenyl)-5-hydroxy-hexahydro-pentalen-2-one **14a**

Magnesium (486 mg, 20 mmol) was added to 20 mL ether and lightly heated. The mixture was stirred and added with catalytic amount iodine and dropwise 1-bromo-4-fluoro-benzene (2.1 mL, 22 mmol) while maintaining the reaction system lightly boiled for 1 hour. The reaction mixture was dropwise added with a solution of 7,7-(ethylidene acetal)bicyclo[3.3.0]octane-3-one **1b** (1.19 g, 6.54 mmol) in 10 mL ether and then was stirred for 2 hours, added with 2 mL 2N hydrochloric acid to stop the reaction for another 2 hours stirring and extracted with ethyl acetate(80 mL×3). The combined organic phase was washed with 15 mL saturated brine, the ethyl acetate was dried over anhydrous sodium sulfate, filtered and concentrated under reduced pressure. The residue was purified by silica gel column chromatography to give the title compound 5-(4-fluoro-phenyl)-5-hydroxy-hexahydro-pentalen-2-one **14a** (900 mg, yield 60%) as a white powder.
MS m/z (ESI) : 235.3[M+1].

Preparation of tert-butyl-[2-(4-carbamoyl-thiazolidin-3-yl)-2-oxo-ethyl] -carbamate **14b**

N-tert-buthylcarbonyl-glycine (0.75 g, 4.31 mmol) and R-thiazolidine-4-carboxamide (1.05 g, 4.31 mmol) were dissolved in 75 mL acetonitrile at 0 , then 1-hydroxybenzotriazole (1.74 g, 12.93 mmol), N-ethyl-N'-(dimethylaminopropane)-carbodiimide (1.65 g, 8.62 mmol) and triethylamine (1.8 mL, 12.93 mmol) were added under stirring. Upon completion of the addition, the reaction mixture was naturally raised to room temperature and stirred for 3 hours. After TLC showed the starting material disappeared, the redundant acenitrile was removed and the reaction mixture was extracted with ethyl acetate (100 mL×3). The combined organic phase was washed with 25 mL saturated brine, dried over anhydrous sodium sulfate, filtered and concentrated under reduced pressure. The residue was purified by silica gel column chromatography to give the title compound tert-butyl-[2-(4-carbamoyl-thiazolidin-3-yl)-2-oxo-ethyl]-carbamate **14b** (1.15 g, yield 92.7%) as a white powder.
MS m/z (ESI) : 290.1[M+1].

Preparation of tert-butyl-[2-(4-cynao-thiazolidin-3-yl)-2-oxo-ethyl] -carbamate **14c**

In a dry three-neck flask under a nitrogen atmosphere, 20 mL pyridine, tert-butyl-[2-(4-carbamoyl-thiazolidin-3-yl)-2-oxo-ethyl]-carbamate **14b** (1.15 g, 3.98 mmol), imidazole (0.57 g, 8.36 mmol) was added in order while maintaining the reaction system at -35 , phosphorus oxychloride (1.52 mL, 16.32 mmol) was added dropwise under stirring for 1 hour at -35 . The reaction mixture was naturally raised to room temperature, aboundent pyridine was evaporated and the reaction mixture was extracted with ethyl acetate (100 mL×3). The combined organic phase was washed with 25 mL saturated brine, dried over anhydrous sodium sulfate, filtered and concentrated under reduced pressure. The residue was purified by silica gel column chromatography to give the title compound tert-butyl-[2-(4-cynao-thiazolidin-3-yl)-2-oxo-ethyl]-carbamate **14c** (0.811 g, yield 75%) as a white powder.
MS m/z (ESI) : 272.3[M+1].

Preparation of 3- {2- [5-(4-Fluoro-phenyl)-5-hydroxy-octahydr-pentalen -2-ylamino]-acetyl}-thiazolidine-2-carbonitrile **14d**

Tert-butyl-[2-(4-cynao-thiazolidin-3-yl)-2-oxo-ethyl]-carbamate **14c** (698 mg, 2.58 mmol) was dissolved in 20 mL dichloromethane, then trifluoroacetic acid (5.93 mL, 77.4 mmol) was added in an ice-water bath. Upon completion of the addition, the reaction mixture was stirred at 0 until reaction was completed. Dichloromethane and trifluoroacetic acid were evaporated. The above residue was dissolved in 15 mL methanol and in order added with triethylamine (1.04 mL, 7.5 mmol), 5-(4-fluoro-phenyl)-5-hydroxy-hexahydro- pentalen-2-one **14a** (600 mg, 2.5 mmol) and sodium triacetoxy borohydride (2.38 g, 11.25 mmol). The reaction mixture was stirred at room temperature, concentrated under reduced pressure and added with 20 mL saturated sodium carbonate solution, then extracted with dichloromethane (80 mL×3). The combined organic phase was washed with 15 mL saturated brine, the dichloromethane was dried over anhydrous sodium sulfate, filtered and concentrated under reduced pressure. The residue was purified by silica gel column chromatography to give the title compound 3-{2- [5-(4-Fluoro-phenyl) -5-hydroxy-octahydro-pentalen-2-ylamino]-acetyl}-thiazolidine -2-carbonitrile **14d** (80mg, yield 19.6%) as a light yellow powder.
MS m/z (ESI) : 390.3[M+1].
$^1$H NMR ( 400 MHz, $CD_3OD$ ) δ( ppm ) 7.35(m, 2H), 6.89(m, 2H), 4.62(m, 1H), 4.39(m, 2H), 3.83(m, 1H), 3.39(m, 1H), 3.18(m, 3H), 3.10(m, 1H), 2.62(m, 2H), 2.06(m, 2H), 2.00-1.72(m, 5H).

Preparation of 3- {2-[5-(4-Fluoro-phenyl)-5-hydroxy-octahydro-pentalen-2-ylamino] -acetyl}-thiazolidine-2-carbonitrile hydrochloride **14**

3-{2-[5-(4-Fluoro-phenyl)-5-hydroxy-octahydro-pentalen-2-ylamino]-acetyl}-thi azolidine-2-carbonitrile **14d** (80 mg, 0.206 mmol) was dispersed in 10 mL ether, then a solution of 0.5N hydrochloric acid in 2 mL ether was added in an ice-water bath. The reaction mixture was evaporated to give the title compound 3-{2-[5-(4-Fluoro-phenyl)-5-hydroxy-octahydro-pentalen-2-ylamino] -acetyl}-thiazolidine-2-carbonitrile hydrochloride **14** (80 mg, yield 91%) as a white powder.
MS m/z (ESI) : 390.2[M+1].

Example 15

Preparation of 1-[2-(5-oxo-octahydro-pentalen-2-ylamino)-acetyl] -pyrrolidine-2-carbonitrile hydrochloride 15

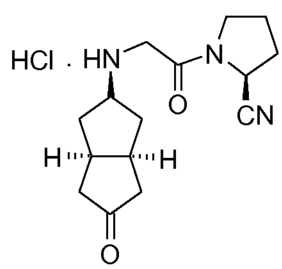

15

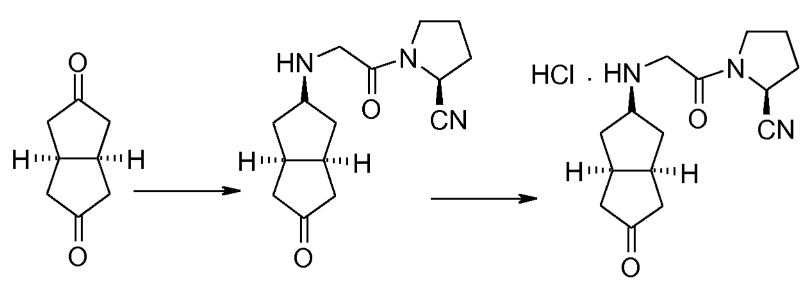

1a 15a 15

Preparation of 1-[2-(5-oxo-octahydro-pentalen-2-ylamino)-acetyl] -pyrrolidine-2-carbonitrile **15a**

[2-(2-Cyano-pyrrolidin-1-yl)-2-oxo-ethyl]-carbamic acid tert-butyl ester (1.83 g, 7.246 mmol) was dissolved in 40 mL dichloromethane, then trifluoroacetic acid (16.65 mL, 217.4 mmol) was added in an ice-water bath. Upon completion of the addition, the reaction mixture was stirred at 0 until reaction was completed. Dichloromethane and trifluoroacetic acid were evaporated. The above residue was dissolved in 40 mL methanol and in order added with triethylamine (3.024 mL, 21.74 mmol), tetrahydro-pentalene-2,5-dione **1a** (1 g, 7.246 mmol) and sodium triacetoxy borohydride (6.142 g, 28.98 mmol). The reaction mixture was stirred overnight at room temperature, concentrated under reduced pressured and added with 10 mL saturated sodium carbonate solution, then extracted with dichloromethane (80 mL×3). The combined organic phase was washed with 15 mL saturated brine, the dichloromethane phase was dried over anhydrous sodium sulfate, filtered and concentrated under reduced pressure. The residue was purified by silica gel column chromatography to give the title compound 1-[2-(5-oxo-octahydro-pentalen -2-ylamino)-acetyl]-pyrrolidine-2-carbonitrile **15a** (100 mg, yield 5%) as a white powder.
MS m/z (ESI) : 276.6[M+1].
$^1$H NMR ( 400 MHz, $CDCl_3$ ) δ( ppm ) 4.69(m, 1H), 3.52(m, 1H), 3.36(m, 1H), 3.16(m, 1H), 2.66(m, 2H), 2.44(m, 3H), 2.24-1.98(m, 8H), 1.25(m, 3H).

Preparation of 1-[2-(5-oxo-octahydro-pentalen-2-ylamino)-acetyl] -pyrrolidine-2-carbonitrile hydrochloride **15**

1-[2-(5-Oxo-octahydro-pentalen-2-ylamino)-acetyl]-pyrrolidine-2-carbonitrile **15a** (100 mg, 0.364 mmol) was dispersed in 10 mL ether, then a solution of 0.5N hydrochloric acid in 2 mL ether was added in an ice-water bath. The resulting solid was centrifuged to give the title compound 1-[2-(5-oxo-octahydro-pentalen -2-ylamino)-acetyl]-pyrrolidine-2-carbonitrile hydrochloride **15** (80 mg, yield 70.1 %) as a white powder.
MS m/z (ESI) : 276.2[M+1].

Example 16

Preparation of 1-[2-(5-hydroxyl-octahydro-pentalen-2-ylamino) acetyl] pyrrolidine-2-carbonitrile hydrochloride 16

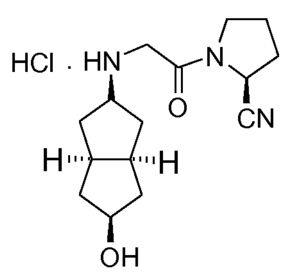

16

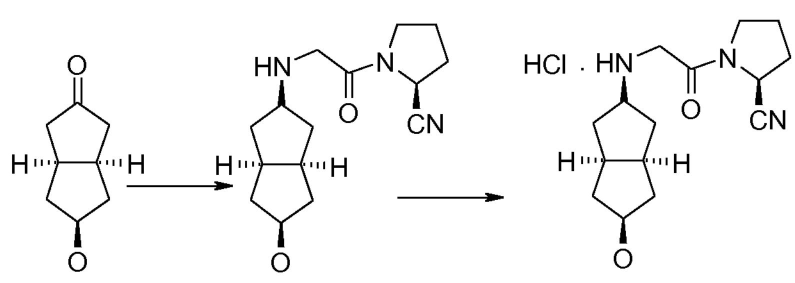

5a 16a 16

Preparation of 1-[2-(5-hydroxyl-octahydro-pentalen-2-ylamino)acetyl] pyrrolidine-2-carbonitrile **16a**

[2-(2-Cyano-pyrrolidin-1-yl)-2-oxo-ethyl]-carbamic acid tert-butyl ester **1f** (903 mg, 3.571 mmol) was dissolved in 40 mL dichloromethane, then trifluoroacetic acid (8.2 mL, 107.14 mmol) was added in an ice-water bath. Upon completion of the addition, the reaction mixture was stirred at 0 until reaction was completed. Dichloromethane and trifluoroacetic acid were evaporated. The above residue was dissolved in 50 mL methanol and in order added with triethylamine (1.49 mL, 10.714 mmol), 5-hydroxy-hexahydro-pentalen-2-one **5a** (0.5 g, 3.571 mmol) and sodium triacetoxy borohydride (3.02 g, 14.285 mmol). The reaction mixture was stirred overnight at room temperature, concentrated under reduced pressure and added with 20 mL saturated sodium carbonate solution, then extracted with ethy acetate (80 mL×3). The combined organic phase was washed with 15 mL saturated brine, the ethyl acetate was dried over anhydrous sodium sulfate, filtered and concentrated under reduced pressure. The residue was purified by silica gel column chromatography to give the title compound 1-[2-(5-hydroxyl-octahydro-pentalen -2-ylamino)acetyl]pyrrolidine-2-carbonitrile **16a**(200 mg, yield 20.4%) as a white powder.
MS m/z (ESI) : 278.5[M+1].
$^1$H NMR ( 400 MHz, $CDCl_3$ ) δ( ppm ) 4.49(m, 1H), 3.98(m, 1H), 3.36(m, 2H), 3.20(m, 1H), 3.07(s, 1H), 2.99(m, 1H), 2.77(m, 2H), 1.98(m, 5H), 1.79(m, 2H), 1.31(m, 3H), 1.05(m, 2H).

Preparation of 1-[2-(5-hydroxyl-octahydro-pentalen-2-ylamino) acetyl] pyrrolidine-2-carbonitrile hydrochloride **16**

1-[2-(5-Hydroxyl-octahydro-pentalen-2-ylamino)acetyl] pyrrolidine-2-carbonitrile **16a** (192 mg, 0.686 mmol) was dispersed in 10 mL ether, then a solution of 0.5N hydrochloric acid in 3 mL ether was added in an ice-water bath. The resulting solid was centrifuged to give the title compound 1-(2-(5-hydroxyl-octahydro-pentalen-2-ylamino)-acetyl) pyrrolidine-2-carbonitrile hydrochloride **16** (80 mg, yield 40%) as a white powder.
MS m/z (ESI) : 278.2[M+1].

Example 17

Preparation of 1-[2-(5-methoxy-octahydro-pentalen-2-ylamino)-acetyl]-pyrrolidine-2-carbonitrile hydrochloride

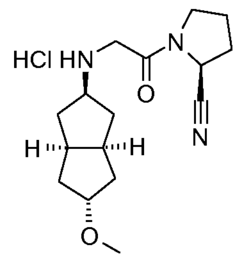

17

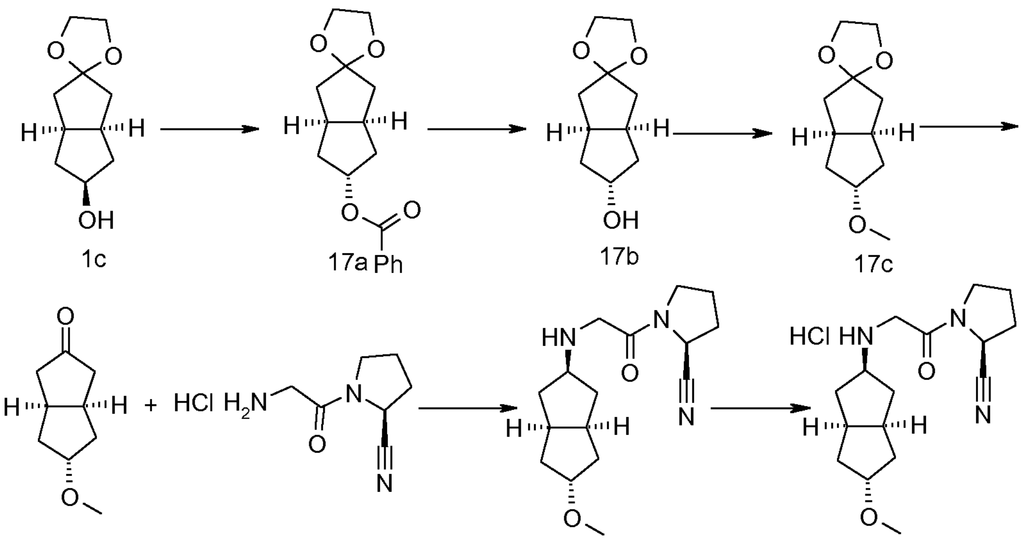

1c 17a 17b 17c

17d 17e 17

Preparation of 7,7-(ethylidene acetal)bicyclo[3.3.0] octane-3-benzoate **17a**

A solution of benzoic acid (2.68 g, 22 mmol) and diethyl azodicarboxylate (3.828 g, 22 mmol) in 4 mL ether was added with a solution of 3-hydroxy-7,7-(ethylidene acetal)bicyclo[3.3.0] octane **1c** (3.68 g, 20 mmol) and triphenylphosphine (5.786 g, 22 mmol) in 50 mL ether. Upon completion of the addition, the reaction mixture was stirred for 14 hours at room temperature, concentrated. The residue was purified by silica gel column chromatography to give the title compound 7,7-(ethylidene acetal)bicyclo[3.3.0] octane-3-benzoate **17a** (2.9 g, yield 50%) as a colorless oil.

Preparation of 3-hydroxy-7,7-(ethylidene acetal)bicyclo[3.3.0] octane **17b**

7,7-(Ethylidene acetal)bicyclo[3.3.0] octane-3-benzoate **17a** (2.9 g, 10.0 mmol) and potassium hydroxide (3.2 g, 22 mmol) were dissolved in a mixture of 73 mL methanol and 37 mL water, the rection mixture was stirred for 2 hours at 40°C. The reaction mixture was extracted with acetic ether (50 mL×4). The combined organic phase was dried over anhydrous sodium sulfate, filtered and concentrated under reduced pressure to give the title compound 3-hydroxy-7,7-(ethylidene acetal)bicyclo[3.3.0] octane **17b**(1.7 g, yield 93%) as a colorless oil.
MS m/z (ESI) : 185.5[M+1].

Preparation of 3-methoxy-7,7-(ethylidene acetal)bicyclo[3.3.0] octane **17c**

3-hydroxy-7,7-(ethylidene acetal)bicyclo[3.3.0] octane **17b** (0.86 g, 4.67 mmol) was dissolved in 50 mL tetrahydrofuran, then sodium hydride(0.45 g, 9.34 mmol) was added. Upon completion of the addition, the reaction mixture was stirred for 1 hour at room temperature, added with methyl iodide (1.162 mL, 18.69 mmol) and heated to reflux for 2 hours, cooled and added with water (10 mL) to stop the reaction, extracted with ethyl acetate (50 mL×4). The combined organic phase was dried over anhydrous sodium sulfate, filtered and concentrated under reduced pressure to give the title compound 3-methoxy-7,7-(ethylidene acetal)bicyclo[3.3.0] octane **17c** to the next step.

Preparation of 5-methoxy- hexahydropentalen-2-one **17d**

3-Methoxy-7,7-(ethylidene acetal)bicyclo[3.3.0] octane **17c** (920mg, 4.67 mmol) was dissolved in the mixture of 50 mL ethyl acetate and 25 mL water, then ethanedioic acid(1.134 g, 9 mmol) was added under stirring. Upon completion of the addition, the reaction mixture was stirred overnight at room temperature, added with 50 mL water, extracted with ethyl acetate (50 mL×3). The combined organic phase was dried over anhydrous sodium sulfate, filtered and concentrated under reduced pressure to give the title compound 5-methoxy- hexahydropentalen-2-one **17d** (0.65 g) as a colorless oil.
MS m/z (ESI) : 157.3[M+1].

Preparation of

1-[2-(5-methoxy-octahydro-pentalen-2-ylamino)-acetyl]-pyrrolidine-2-carbonitrile **17e**

5-Methoxy- hexahydropentalen-2-one **17d** (308 mg, 2 mmol) was dissolved in 50 mL tetrahydrofuran, then 1-(2-Amino-acetyl)-pyrrolidine-2-carbonitrile hydrochloride (630 mg, 3.32 mmol) was added. The reaction mixture was stirred for 0.5 hour, and then added with sodium sulfate (5 g), sodium triacetoxy borohydride (1.4 g, 6.6 mmol). Upon completion of the addition, the reaction mixture was stirred for 3 hours at room temperature, added with 20 mL saturated sodium carbonate solution, extracted with ethyl acetate (50 mL×5), extracted with dichloromethane (50 mL×10). The combined organic phase was washed with 50 mL saturated brine, dried over anhydrous sodium sulfate, filtered and concentrated under reduced pressure. The residue was purified by silica gel column chromatography to give the title compound 1-[2-(5-methoxy-octahydro-pentalen-2-ylamino)-acetyl]-pyrrolidine-2-carbonitrile **17e** (250 mg, yield 43%) as a colorless oil.
MS m/z (ESI) : 320.3[M+1].

Preparation of

1-[2-(5-methoxy-octahydro-pentalen-2-ylamino)-acetyl]-pyrrolidine-2-carbonitrile hydrochloride **17**

1-[2-(5-Methoxy-octahydro-pentalen-2-ylamino)-acetyl]-pyrrolidine-2-carbonitrile **17e** (250 mg, 0.313 mmol) was dispersed in 10 mL ether, then a 2 mL solution of 0.5N hydrochloric acid in ether was added in an ice-water bath. The resulting solid was centrifuged to give the title compound 1-[2-(5-methoxy-octahydro-pentalen-2-ylamino)-acetyl]-pyrrolidine-2-carbonitrile hydrochloride **17** (250 mg, yield 76.3%) as a white powder.
MS m/z (ESI) : 292.6[M+1].

Example 18

Preparation of 1-[2-(5-methoxy-octahydro-pentalen-2-ylamino)-acetyl)] pyrrolidine-2-carbonitrile hydrochloride 18

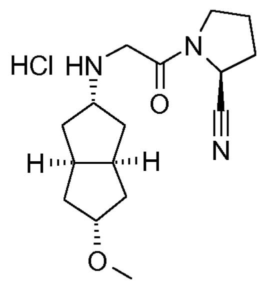

18

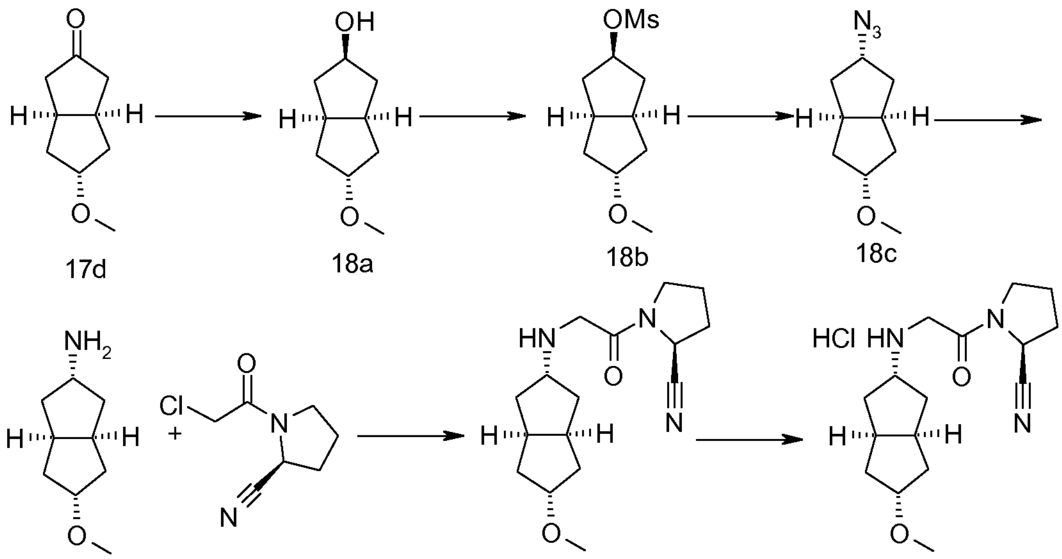

17d 18a 18b 18c

18d 18e 18

Preparation of 5-methoxy-octahydro-pentalen-2-ol **18a**

5-Methoxy- hexahydropentalen-2-one **17d** (2.5 g, 7.12 mmol) was dissolved in 50 mL methanol, then sodium borohydride (0.537g, 14.2 mmol) was added in an ice-water bath. Upon completion of the addition, the reaction mixture was stirred for 30 min, and then added with 10 mL water and small volume acetone (2 mL) to stop the reaction, concentrated under reduced pressure, extracted with acetic ether (50 mL×3). The combined organic phase was dried over anhydrous sodium sulfate, filtered and concentrated under reduced pressure to give the title compound 5-methoxy-octahydro-pentalen-2-ol **18a** (1.46 g) as a colorless oil to be directly used in the further reaction.

Preparation of methanesulfonic acid 5-methoxy-octahydro-pentalen-2-yl ester **18b**

5-Methoxy-octahydro-pentalen-2-ol **18a** (1.47 g, 7.0 mmol) was dissolved in 50 mL dichlormethane, and triethylamine (1.95 ml, 14.0 mmol) was added, then methanesulfonyl chloride (0.7 ml, 9.1 mmol) was added in an ice-water bath. Upon completion of the addition, the reaction mixture was stirred overnight, added with 50 mL water, extracted with dichlormethane (50 mL×4). The combined organic phase was dried over anhydrous sodium sulfate, filtered and concentrated under reduced pressure, the residue was purified by silica gel column chromatography to give the title compound methanesulfonic acid 5-methoxy-octahydro-pentalen-2-yl ester **18b** (1.13 g, yield 69.0%) as a colorless oil.

Preparation of 5-methoxy-octahydro-pentalen-2-yl azide group **18c**

Methanesulfonic acid 5-methoxy-octahydro-pentalen-2-yl ester **18b** (1.13 g, 4.82 mmol), sodium azide (0.313 g, 4.82 mmol) were dissolved in 50 mL N,N-dimethylformamide under an argon atmosphere, the reaction mixture was heated to 65˚C overnight. The resulting mixture was cooled, concentrated, added with 50 mL wate, then extracted with ethyl acetate (50 mL×4) and. The combined organic phase was dried over anhydrous sodium sulfate, filtered and concentrated under reduced pressure. The residue was purified by silica gel column chromatography to give 5-methoxy-octahydro-pentalen-2-yl azide group **18c** (500 mg, yield 57%) as a colorless oil.

Preparation of 5-methoxy-octahydro-pentalen-2-ylamine **18d**

5-Methoxy-octahydro-pentalen-2-yl azide group **18c** (0.15 g, 0.83 mmol) was dissolved in 50 mL methanol, Pd-C (0.05 g) and chloroform (0.1 ml) was added. Upon completion of the addition, the reaction mixture was hydrogenated for 3 hours under 0.3Mpa at room temperature, filtered and washed with methanol (10 mLx5). The filtrate was evaporated to give the title compound 5-methoxy-octahydro-pentalen-2-ylamine **18d** (0.16 g) to be used directly in the next step.
MS m/z (ESI) : 156.5[M+1].

Preparation of 1-[2-(5-methoxy-octahydro-pentalen-2-ylamino)-acetyl] -pyrrolidine-2-carbonitrile **18e**

5-Methoxy-octahydro-pentalen-2-ylamine **18d** (0.16 g, 0.83 mmol), 1-(2-chloro-acetyl)-pyrrolidine-2-carbonitrile (143 mg, 0.83 mmol) [J. Med. Chem. 2002 45(12), 2362-2365], potassium carbonate(0.230 g, 1.66 mmol) and catalytic amount potassium iodide were dissolved in 100 mL acetonitrile. Upon completion of the addition, the reaction mixture was stirred overnight at room temperature, added with 20 mL water, adjusted to pH >10 with 1N sodium hydroxide solution and extracted with ethyl acetate (50 ml×8) while maintaining pH >10 in water phase. The combined organic phase was washed with saturated brine (50 mL), dried over anhydrous sodium sulfate, filtered and concentrated under reduced pressure. The residue was purified by silica gel column chromatography to give the title compound 1-[2-(5-methoxy-octahydro-pentalen-2-ylamino)-acetyl]-pyrrolidine-2-carbonitrile **18e** (0.142 g) as a colorless oil to be directly used in the further step.
MS m/z (ESI) : 292.6[M+1].

Preparation of 1-[2-(5-methoxy-octahydro-pentalen-2-ylamino)-acetyl] -pyrrolidine-2-carbonitrile hydrochloride **18**

1-[2-(5-Methoxy-octahydro-pentalen-2-ylamino)-acetyl]-pyrrolidine-2-carbonitri le **18e** (142 mg, 0.487 mmol) was dispersed in 10 mL ether, then a 2 mL solution of 0.5N hydrochloric acid in ether was added in an ice-water bath. The resulting solid was centrifuged to give the title compound 1-[2-(5-methoxy-octahydro-pentalen-2-ylamino)-acetyl]-pyrrolidine-2-carbonitrile hydrochloride **18** (142 mg, yield 89%) as a white powder.

Example 19

Preparation of 1-[2-(5-methoxy-octahydro-pentalen-2-ylamino)-acetyl] -pyrrolidine-2-carbonitrile hydrochloride **19**

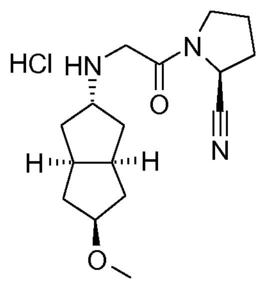

19

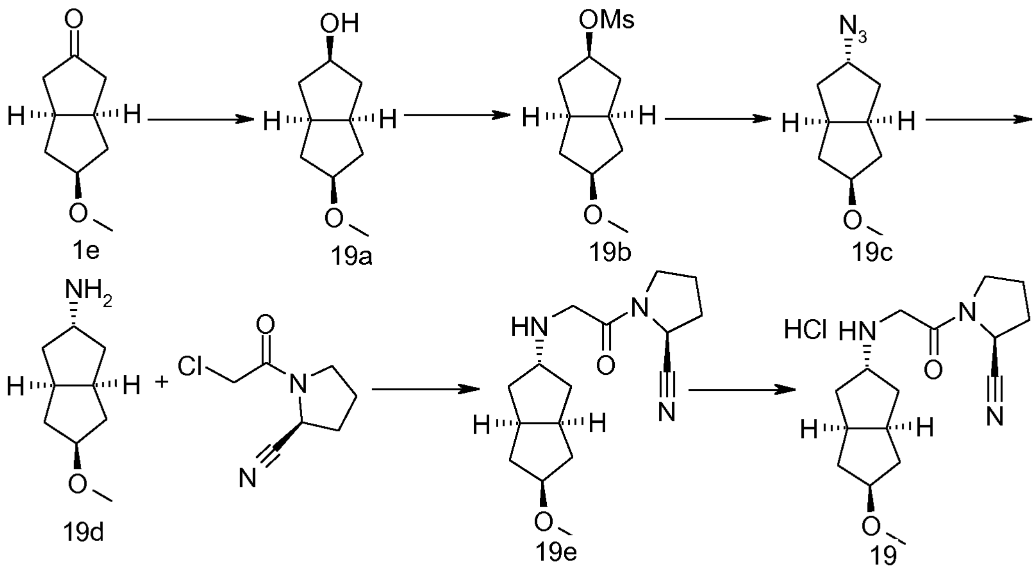

1e 19a 19b 19c

19d 19e 19

Preparation of 5-methoxy-octahydro-pentalen-2-ol **19a**

5-Methoxy-hexahydropentalen-2-one **1e** (2.5 g, 7.12 mmol) was dissolved in 50 mL methanol, then sodium borohydride (0.537 g, 14.2 mmol) was added in an ice-water bath. Upon completion of the addition, the reaction mixture was stirred for 30 min, then added with 10 mL water and small volume acetone (2 mL) to stop the reaction, concentrated under reduced pressure, extracted with acetic ether (50 mL×3). The combined organic phase was dried over anhydrous sodium sulfate, filtered and concentrated under reduced pressure to give the title compound 5-methoxy-octahydro-pentalen-2-ol **19a** (1.46 g) as a colorless oil to be directly used in the further reaction.

Preparation of methanesulfonic acid 5-methoxy-octahydro-pentalen-2-yl ester **19b**

5-Methoxy-octahydro-pentalen-2-ol **19a** (1.46 g, 7.12 mmol) was dissolved in 50 mL dichlormethane, and triethylamine (1.98 ml, 14.2 mmol) was added, then methanesulfonyl chloride (0.66 ml, 8.54 mmol) was added in an ice-water bath. Upon completion of the addition, the reaction mixture was stirred overnight, added with 50 mL water, extracted with dichlormethane (50 mL×4). The combined organic phase was dried over anhydrous sodium sulfate, filtered and concentrated under reduced pressure, the residue was purified by silica gel column chromatography to give the title compound methanesulfonic acid 5-methoxy-octahydro-pentalen-2-yl ester **19b** (1.47 g, yield 88.1%) as a colorless oil.

Preparation of 5-methoxy-octahydro-pentalen-2-yl azide group **19c**

Methanesulfonic acid 5-methoxy-octahydro-pentalen-2-yl ester **19b** (1.47 g, 6.27 mmol), sodium azide (0.408 g, 6.27 mmol) were dissolved in 50 mL N,N-dimethylformamide under an argon atmosphere, the reaction mixture was heated to 65°C overnight. The resulting mixture was cooled, concentrated under reduced pressure, added with 50 mL water and extracted with ethyl acetate (50 mL×4). The combined organic phase was dried over anhydrous sodium sulfate, filtered and concentrated under reduced pressure. The residue was purified by silica gel column chromatography to give the title compound 5-methoxy-octahydro-pentalen-2-yl azide group **19c** (540 mg, yield 48%) as a colorless oil.

Preparation of 5-methoxy-octahydro-pentalen-2-ylamine **19d**

5-Methoxy-octahydro-pentalen-2-yl azide group **19c** (0.52 g, 2.87 mmol) was dispersed in 20 mL methanol, then Pd-C (0.25 g) was added. Upon completion of the addition, the reaction mixture was hydrogenated for 3 hours under 0.3Mpa at room temperature, filtered and Pd-C was washed with methanol(10 mL×5). The filtrate was concentrated to give the title compound 5-methoxy-octahydro-pentalen-2-ylamine **19d** (0.42 g, yield 92.2%).
MS m/z (ESI) : 156.5[M+1].

Preparation of 1-[2-(5-methoxy-octahydro-pentalen-2-ylamino)-acetyl] -pyrrolidine-2-carbonitrile **19e**

5-Methoxy-octahydro-pentalen-2-ylamine **19d** (0.42 g, 1.3 mmol), 1-(2-chloro-acetyl)-pyrrolidine-2-carbonitrile (334 mg, 1.9 mmol) [J. Med. Chem. 2002 45(12), 2362-2365], potassium carbonate(0.719 g, 5.2 mmol) and potassium iodide(100 mg, 0.6mmol) was dissolved in 20 mL dichlormethane. Upon completion of the addition, the reaction mixture was stirred overnight at room temperature, added with 20 mL water, adjusted to pH >10 with 1N sodium hydroxide solution and extracted with ethyl acetate (50 mlx8) while maintaining pH >10 in water phase. The combined organic phase was washed with saturated brine (50 mL), dried over anhydrous sodium sulfate, filtered and concentrated under reduced pressure. The residue was purified by silica gel column chromatography to give the title compound 1-[2-(5-methoxy-octahydro-pentalen-2-ylamino)-acetyl]-pyrrolidine-2-carbonitrile **19e** (0.2 g, yield 35%) as a colorless oil.
MS m/z (ESI) : 292.6[M+1].

Preparation of 1-[2-(5-methoxy-octahydro-pentalen-2-ylamino)-acetyl] -pyrrolidine-2-carbonitrile hydrochloride **19**

1-[2-(5-Methoxy-octahydro-pentalen-2-ylamino)-acetyl]-pyrrolidine-2-carbonitri le **19e** (200 mg, 0.687 mmol) was dispersed in 10 mL ether , then a 2 mL solution of 0.5N hydrochloric acid in ether was added in an ice-water bath. The resulting solid was centrifuged to give the title compound 1-[2-(5-methoxy-octahydro-pentalen-2-ylamino)-acetyl]-pyrrolidine-2-carbonitrile hydrochloride **19** (180 mg, yield 80%) as a white powder.

## BIOLOGICAL ASSAYS

### Active Inhibition DPP IV Assay

Aim directly to target DPP IV, Detection method is chemical Luminescent assay, using DPP IV-Glo™ Protease Assay Kit (cat. G8350) of Promega company and Dipeptidylpeptidase IV, Human Placenta (cat. 317630) of Calbiochem company. According to the manual of the kit, this invention inspected the amout of the each reagent in the experimental, corresponding amount of DPPIV enzyme and chemiluminescence detection methods.
The invention inspected the $IC_{50}$ of the test samples for two parallel treatment, the inhibition rate of compounds for DPP IV is measured and showed in table 1.

**Table 1 Compounds for DPP IV inhibition assay results**

| Example | Structure | $IC_{50}$(μM) |
|---|---|---|
| 2 | 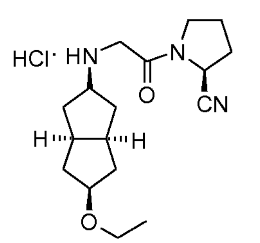 | 0.1269 |

(continued)

| Example | Structure | $IC_{50}$(μM) |
|---|---|---|
| 3 | 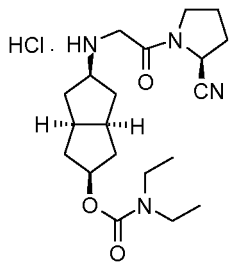 | 0.0405 |
| 4 | 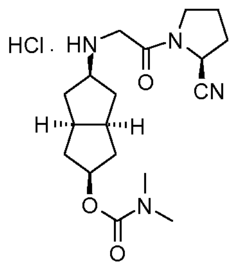 | 0.0613 |
| 5 | 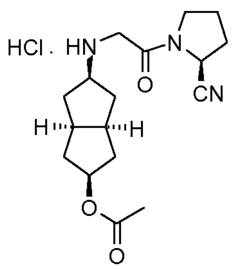 | 0.0322 |
| 6 | 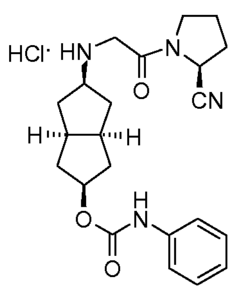 | 0.1211 |
| 7 | 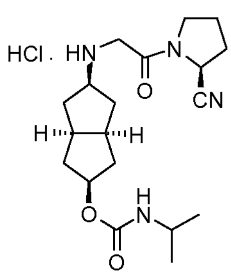 | 0.2036 |

(continued)

| Example | Structure | $IC_{50}$(μM) |
|---|---|---|
| 8 | 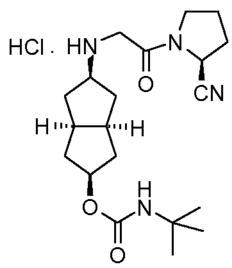 | 0.3257 |
| 10 | 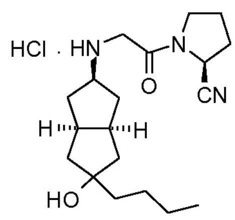 | 0.0183 |
| 14 | 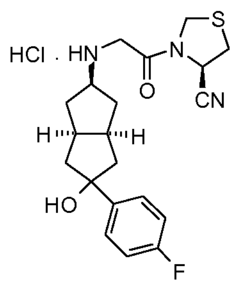 | >2 |

**Preliminary evaluation on hypoglycemic activity of DPPIV inhibitors**

Effects on glucose tolerance in normal ICR mice of examples 15 and 16 were determined. Hypoglycemic activities in vivo were preliminary evaluated.

**Test drugs:**

Preparation procedure: drugs were accurately weighed and dissolved in double-distilled water, misce bene to obtain 0.5mg/ml suspension, then diluted into colorless and clear solutions which concentration were 0.15, 0.05 and 0.015mg/ml.
Administrated Dose: the oral doses were 0.3, 1,3, 10 mg/kg and the volume was 20ml/kg.

**Positive control:**

Name: LAF-237
Preparation procedure: drugs were accurately weighed and dissolved in double-distilled water, misce bene to obtain 0.5mg/ml suspension, then diluted into colorless and clear solutions which concentration were 0.15, 0.05 and 0.015mg/ml. The oral doses were 0.3, 1,3, 10 mg/kg and the volume was 20ml/kg.

**Serum glucose determination:**

Glucose kits was used in the determination of serum glucose levels. Take 250 μl enzyme fluid, then add 5 μl

serum. Establish blank tube (by added 5 μl double-distilled) and the standard tube (by added 5 μl standard glucose solution) simultaneously, misce bene. 37°C water bath for 20min. To adjust to zero value with blank tube, OD(505nm) levels were determined.

$$\text{Serum glucose level BG(mmol/l)}=OD_{\text{sample tube}}/OD_{\text{standard tube}}\times 5.55.$$

**Conclusion:**

It was found that both examples 15 and 16 have good inhibitor activities of DPPIV in vivo, wherein example 16 is slightly better than LAF-237.

## Claims

**1.** Compounds of a Formula (I) or pharmaceutically acceptable salts thereof:

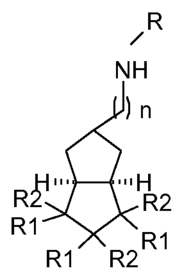

(I)

wherein:

R is selected from the group consisting of alkyl, cycloalkyl, haloalkyl, aryl, heteroaryl, aminocarbonyl alkyl, amide alkyl, heterocyclo aminocarbonyl alkyl and aminoalkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;

$R_1$ and $R_2$ are each independently selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

wherein $R_1$ and $R_2$ are attached together with the atom to form a 3 to 8 membered ring, wherein the 3 to 8 membered hetero ring further contains one or more heteroatoms selected from the group consisting of N, O and S, and the 3 to 8 membered hetero rings so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting

of N, O and S, and the 3 to 8 membered rings so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
n is an integral from 0 to 4;
r is an integral from 1 to 6;
m is an integral from 0 to 6.

**2.** The compounds or pharmaceutically acceptable salts thereof of claim 1, wherein the compounds include the Formula (IA) :

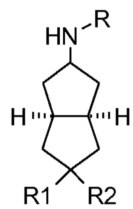

(IA)

wherein:

R is selected from the group consisting of alkyl, cycloalkyl, haloalkyl, aryl, heteroaryl, aminocarbonyl alkyl, amide alkyl, heterocyclo aminocarbonyl alkyl and aminoalkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;
$R_1$ and $R_2$ are each independently selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;
wherein $R_1$ and $R_2$ are attached together with the atom to form a 3 to 8 membered ring, wherein the 3 to 8 membered hetero ring further contains one or more heteroatoms selected from the group consisting of N, O and S, and the 3 to 8 membered hetero rings so formed are each substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;
meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero ring so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
r is an integral from 1 to 6;
m is an integral from 0 to 6.

**3.** The compounds or pharmaceutically acceptable salts thereof of claim 1, wherein:

R is heterocyclo aminocarbonyl alkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;
$R_1$ is hydrogen or hydroxyl;
$R_2$ is selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups consisting of alkyl, halogen, aryl, hydroxyl, amino, alkylamino, amide group, alkoxyl, aryloxyl, heterocylco alkyl, carboxylic acid and carboxylic ester;
$R_4$ and $R_5$ are each independently selected from the group consisting of hydrogen, alkyl, cycloalkyl, aryl, heteroaryl and heterocyco alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;
meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
r is an integral from 1 to 6;
m is an integral from 0-6.

**4.** The compounds or pharmaceutically acceptable salts thereof of claim 1, wherein R is the formula:

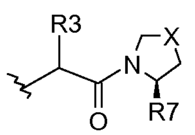

wherein $R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;
$R_7$ is selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heteroalkyl, carboxylic acid, carboxylic ester and halogen;
X is C, S or O.

**5.** The compounds or pharmaceutically acceptable salts thereof of claim 1, wherein n is 0.

**6.** The compounds or pharmaceutically acceptable salts thereof of claim 1, wherein the compounds of formula (I) are in the pharmaceutically acceptable free-form and the forms of acid addition salts, wherein the salts comprise the salts formed with the acids selected from the group consisting of hydrochloric acid, methanesulfonic acid, sulfuric acid, phosphoric acid, citric acid, acetic acid and trifluoroacetic acid.

**7.** The compounds or pharmaceutically acceptable salts thereof of claim 6, wherein the acids are hydrochloric acid or trifluoroacetic acid.

**8.** A preparation process of the compounds of formula (I) of claim 1, wherein the preparation process comprises the steps of:

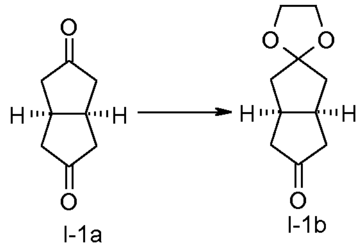

I-1a I-1b

reacting starting material tetrahydro-pentalene-2,5-dione(I-1a) with ethylene glycol and a catalyst of p-toluenesulfonic acid through refluxing in the solvent of benzene to give 7,7-(ethylidene acetal)bicyclo[3.3.0] octane-3-one protected (I-1b);

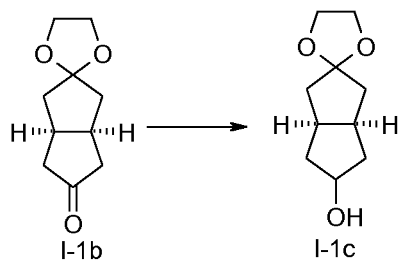

I-1b I-1c

reducing the obtained 7,7-(ethylidene acetal)bicyclo[3.3.0] octane-3-one (I-1b) by $NaBH_4$ at room temperature to afford 3-hydroxy-7,7-(ethylidene acetal)bicyclo[3.3.0] octane (I-1c);

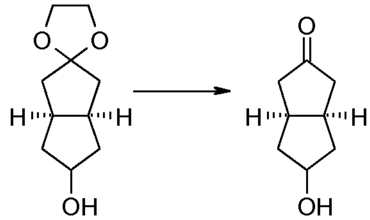

I-1c I-1e

reacting the obtained 3-hydroxy-7,7-(ethylidene acetal)bicyclo[3.3.0] octane (I-1c) with oxalic acid in the mixed solvent of ethyl acetate and water to give 5-hydroxy-hexahydro-pentalen-2-one (I-1e);

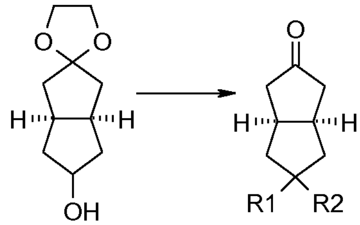

I-1c I-1d

or reacting the obtained 3-hydroxy-7,7-(ethylidene acetal)bicyclo[3.3.0] octane (I-1c) with different isocyanate and trimethylchlorosilane at room temperature or with different Grignard reagent in the solvent of ether and then acidifying it further by 2N hydrochloric acid to give the compounds of the formula (I-1d);

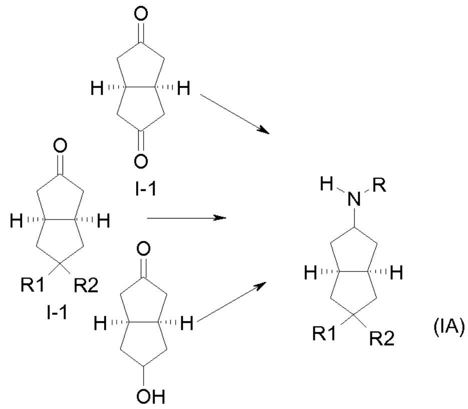

I-1

I-1

I-1

(IA)

reacting the compounds of formula (I-1a) or formula (I-1d) or formula (I-1e) each independently with equivalent different amine in the solvent of methanol under the presence of sodium triethoxyborohydride and triethylamine at room temperature to give the compounds of formula (IA); wherein:

R is selected from the group consisting of alkyl, cycloalkyl, haloalkyl, aryl, heteroaryl, aminocarbonyl alkyl, amide alkyl, heterocyclo aminocarbonyl alkyl and aminoalkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;
$R_1$ and $R_2$ are each independently selected from the group consisting of hydrogen, hydroxyl, alkyl, cycloalkyl, heterocyclo alkyl, aryl, heteroaryl, $-OR_4$, $-(CH_2CH_2O),R_6$, $-(CH_2)_m,C(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;
wherein $R_1$ and $R_2$ are attached together with the atom to form a 3 to 8 membered ring, wherein the 3 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;
meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;
$R_6$ is selected from the group consisting of hydrogen or alkyl;
r is an integral from 1 to 6;
m is an integral from 0 to 6.

**9.** The preparation process of claim 8, wherein:

R is heterocyclo aminocarbonyl alkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;

$R_1$ is hydrogen or hydroxyl, and $R_2$ is selected from the group consisting of hydrogen, hydroxyl, alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_6$ is selected from the group consisting of hydrogen or alkyl;

r is an integral from 1 to 6;

m is an integral from 0 to 6.

**10.** The preparation process of claim 8, wherein R is the formula

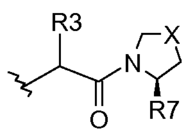

wherein, $R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester;

$R_7$ is selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heteroalkyl, carboxylic acid, carboxylic ester and halogen;

X is C, S or O.

**11.** The preparation process of claim 8, wherein the preparation process also comprises that the obtained compounds of formula (IA) through purification are directly reacted with acids in the solvent of ether under an ice-water bath to give the acid addition salt thereof.

**12.** The preparation process of claim 8, wherein the preparation process also comprises that the obtained compounds of formula (IA) is reacted with di-*tert*-butyl dicarbonate to protect nitrogen atom, purified the compounds by silica gel column chromatography, then reacted with acid in the solvent of ether under an ice-water bath to give the acid addition salt thereof.

**13.** The preparation process of claims 11 or 12, wherein the acids are hydrochloric acid, methanesulfonic acid, sulfuric acid, phosphoric acid, citric acid, acetic acid or trifluoroacetic acid.

**14.** The preparation process of claims 11 or 12, wherein the acids are hydrochloric acid or trifluoroacetic acid.

**15.** The preparation process of claim 8, wherein the compounds of formula (I-1a) or formula (I-1d) or formula (I-1e) is

each independently reacted with equivalent $RNH_2$ in the solvent of methanolunder the presence of sodium triethoxyborohydride and triethylamine at room temperature to give the compounds of formula (IA); wherein
R is selected from the group consisting of alkyl, cycloalkyl, haloalkyl, aryl, heteroaryl, aminocarbonyl alkyl, amide alkyl, heterocyclo aminocarbonyl alkyl and aminoalkyl, wherein the heterocycle is selected from the group consisting of 5- or 6-membered hetero ring further substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester and halogen;

**16.** The preparation process of claim 15, wherein R is the formula

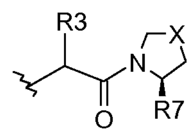

wherein, $R_3$ is selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, carboxylic acid and carboxylic ester; $R_7$ is selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heteroalkyl, carboxylic acid, carboxylic ester and halogen;
X is C, S or O.

**17.** The compounds or pharmaceutically acceptable salts thereof of claim 1, wherein the compounds are selected from the group consisting of:

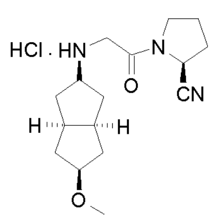
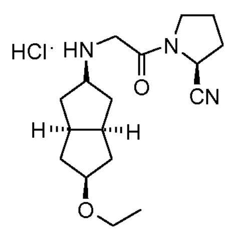
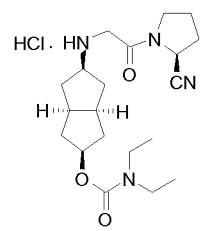

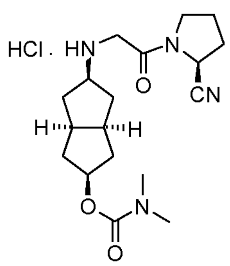
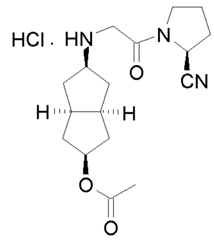
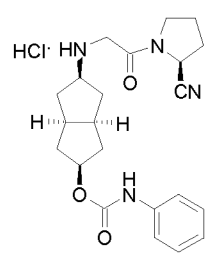

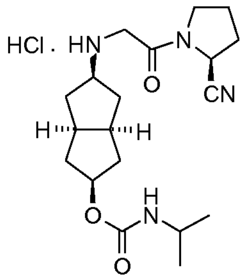
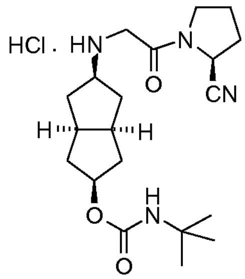
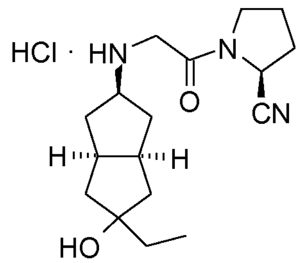
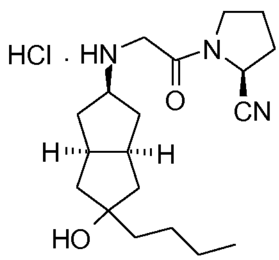
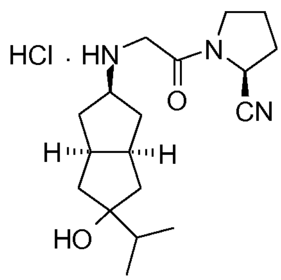
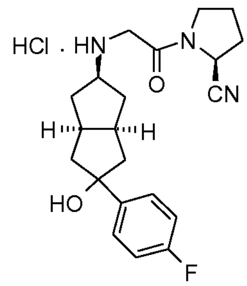
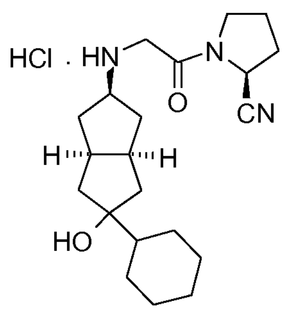
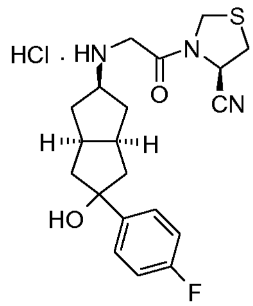
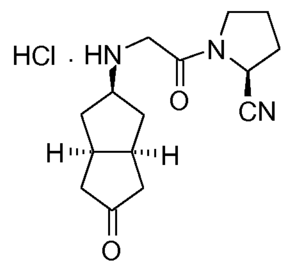
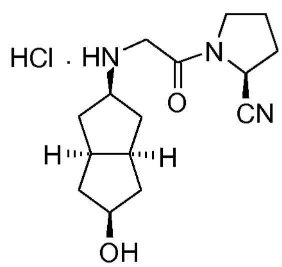
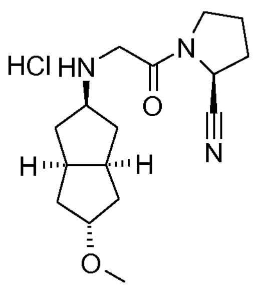
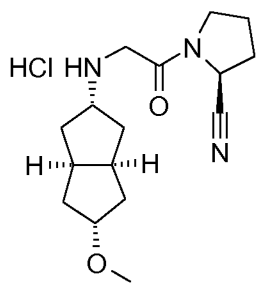

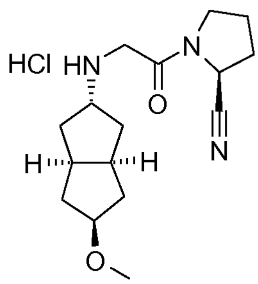

**18.** Compounds as intermediates in the synthesis of compounds of claim 1 having the following formula (I-1d) or (I-1e):

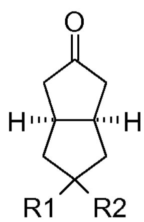
(I-1d)

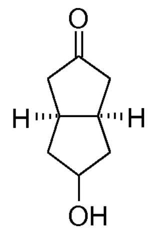
(I-1e)

wherein:

$R_1$ and $R_2$ are each independently selected from the group consisting of alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ or $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocycloalkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_6$ is selected from the group consisting of hydrogen or alkyl;

r is an integral from 1 to 6;

m is an integral from 0 to 6.

**19.** A preparation process of the compounds of formula (I-1d) or (I-1e) of claim 18: comprising the following steps of:

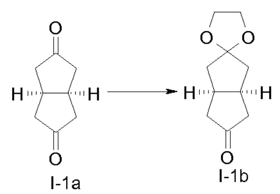
I-1a I-1b

reacting starting material tetrahydro-pentalene-2,5-dione(I-1a) with ethylene glycol and a catalyst of p-toluenesulfonic acid through refluxing in the solvent of benzene to give 7,7-(ethylidene acetal)bicyclo[3.3.0] octane-3-one protected (I-1b);

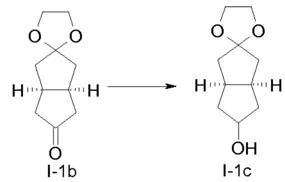

I-1b I-1c

reducing the obtained 7,7-(ethylidene acetal)bicyclo[3.3.0] octane-3-one (I-1b) by $NaBH_4$ at room temperature to afford 3-hydroxy-7,7-(ethylidene acetal)bicyclo[3.3.0] octane (I-1c);

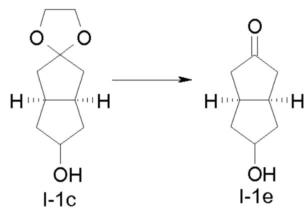

I-1c I-1e

reating the obtained 3-hydroxy-7,7-(ethylidene acetal)bicyclo[3.3.0] octane (I-1c) with oxalic acid in the mixed solvent of ethyl acetate and water to give 5-hydroxy-hexahydro-pentalen-2-one (I-1e);

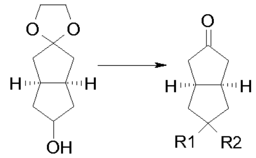

I-1c I-1d

or reacting the obtained 3-hydroxy-7,7-(ethylidene acetal)bicyclo[3.3.0] octane (I-1c) with different isocyanate and trimethylchlorosilane at room temperature or with different Grignard reagent in the solvent of ether and then acidifying it further by 2N hydrochloric acid to give the compounds of the formula (I-1d);
wherein:

$R_1$ and $R_2$ are each independently selected from the group consisting of alkyl, cycloalkyl, heterocyclo alkyl, aryl, heteroaryl, $-OR_4$, $-(CH_2CH_2O)_rR_6$, $-(CH_2)_mC(O)OR_4$, $-(CH_2)_mC(O)NR_4R_5$, $-(CH_2)_mOC(O)NR_4R_5$, $-C(O)R_4$, $-NR_6C(O)R_5$, $-NR_4C(O)OR_5$, $-OC(O)OR_4$, $-OC(O)NR_4R_5$, $-NC(O)NR_4R_5$ and $-NR_4R_5$, wherein the alkyl, cycloalkyl, heterocyclo alkyl, aryl or heteroaryl is further substituted by one or more groups selected from the group consisting of alkyl, halogen, aryl, hydroxyl, amino, alkyl amino, amide group, alkoxyl, aryloxyl, heterocyclo alkyl, carboxylic acid and carboxylic ester;

$R_4$ and $R_5$ are each independently selected from the group consisting of hydrogen, alkyl, cycloalkyl, aryl, heteroaryl and heterocyclo alkyl, wherein the alkyl, cycloalkyl, aryl, heteroaryl or heterocyclo alkyl is further substituted by one or more groups selected from the group consisting of alkyl, cycloalkyl, aryl, heteroaryl, alkoxyl, cycloalkoxyl, aryloxyl, heteroaryloxyl, halogen, hydroxyl, amino, alkylamino, amide group, aminocarbonyl, cyano, aminoalkyl, hydroxyalkyl, heterocyclo alkyl, heterocyclo alkoxyl, trifluoromethyl, carboxylic acid and carboxylic ester;

meanwhile, $R_4$ and $R_5$ are attached together with the N atom to form the 3 to 8 membered hetero ring, wherein the 5 to 8 membered hetero ring further contains one or more heteroatoms selected from the group consisting of N, O and S, and the 3 to 8 membered hetero rings so formed are substituted by one or more groups selected from the group consisting of alkyl, aryl, heteroaryl, haloalkyl, haloalkoxyl, hydroxyl, amino, alkylamino, amide group, aminocarbonyl, cyano, alkoxyl, aryloxyl, aminoalkyl, hydroxyalkyl, heterocyclo alkyl, carboxylic acid, carboxylic ester, halogen and $-NR_4R_5$;

$R_6$ is selected from the group consisting of hydrogen or alkyl;
r is an integral from 1 to 6;
m is an integral from 0 to 6.

**20.** A pharmaceutical composition comprising a compound or salt thereof in an effective therapeutic dose of any one of claims 1-7, 17, as well as pharmaceutically acceptable carrier.

**21.** Use of the compounds or salts thereof of claim 1 in the preparation of a medicament as a dipeptidyl peptidase IV (DPPIV) inhibitor.

**22.** Use of the composition of claim 20 in the preparation of a medicament as a dipeptidyl peptidase IV (DPPIV) inhibitor.

## INTERNATIONAL SEARCH REPORT

International application No. PCT/CN2007/001008

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)

IPC[8] C07C, C07D, A61K, A61P

Documentation searched other than minimum documentation to the extent that such documents are included in the fields searched

Electronic data base consulted during the international search (name of data base and, where practicable, search terms used)

WPI, EPODOC, PAJ, CPRS, CNKI: bicyclooctane, cyclopentadienyl, ethylamine, diabetes, DPPIV

C. DOCUMENTS CONSIDERED TO BE RELEVANT

| Category* | Citation of document, with indication, where appropriate, of the relevant passages | Relevant to claim No. |
|---|---|---|
| X | Sundby, Eirik,et al." 2,2-Dimethyl-1,3-propanediol as protective group promotes microbial hydroxylation of cis-bicyclo[3.3.0]octane-3,7-dione", Biotechnology Letters (1998), 20(4), 337-340, compound 7 | 18 |
| A | CN1639159A (ARANYL P) 13.Jul 2005 (13.07.2005) the whole document | 1-22 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 25.May 2007(25.05.2007) | **30.AUG 2007 (30.08.2007)** |

Name and mailing address of the ISA/CN
The State Intellectual Property Office, the P.R.China
6 Xitucheng Rd., Jimen Bridge, Haidian District, Beijing, China
100088
Facsimile No. 86-10-62019451

Authorized officer
Lvqing
Telephone No. (86-10)62085607

Form PCT/ISA /210 (second sheet) (April 2007)

## INTERNATIONAL SEARCH REPORT

International application No.
PCT/CN2007/001008

Continuation of: CLASSIFICATION OF SUBJECT MATTER

C07D207/16(2006.01)i

C07D275/03(2006.01)i

A61K31/426(2006.01)i

A61P3/10(2006.01)i

C07C49/345(2006.01)n

Form PCT/ISA /210 (extra sheet) (April 2007)

**INTERNATIONAL SEARCH REPORT**
Information on patent family members

International application No.
PCT/CN2007/001008

| Patent Documents referred in the Report | Publication Date | Patent Family | Publication Date |
|---|---|---|---|
| CN1639159A | 13.07.2005 | WO03074500A2 | 12.09.2003 |
| | | AU2003209514A1 | 16.09.2003 |
| | | HU0200849A1 | 30.08.2004 |
| | | EP1487807A2 | 22.12.2004 |
| | | KR20040091102A | 27.10.2004 |
| | | BRPI0307960A | 15.02.2005 |
| | | NO20044221A | 06.12.2004 |
| | | US2005130981A1 | 16.06.2005 |
| | | TW200303860A | 16.09.2003 |
| | | ZA200406467A | 31.08.2005 |
| | | JP2005529078T | 29.09.2005 |
| | | INKOLNP200401079E | 27.01.2006 |

Form PCT/ISA /210 (patent family annex) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9819998 A
- US 6110949 A


### Non-patent literature cited in the description


- *Endocrinology,* 1999, vol. 140, 5356-5363
- *J. Med. Chem.,* 2002, vol. 45 (12), 2362-2365